(19) Europäisches Patentamt
European Patent Office
Office européen des brevets

(11) EP 4 203 919 B1

(12) **EUROPEAN PATENT SPECIFICATION**

(45) Date of publication and mention of the grant of the patent:
**31.01.2024 Bulletin 2024/05**

(21) Application number: **22760986.4**

(22) Date of filing: **05.08.2022**

(51) International Patent Classification (IPC):
***A61K 9/107*** (2006.01) ***A61K 9/48*** (2006.01)
***A61K 31/4709*** (2006.01) ***A61P 7/10*** (2006.01)

(52) Cooperative Patent Classification (CPC):
**A61K 9/1075; A61K 9/107; A61K 9/4825; A61K 9/4866; A61K 31/4709**

(86) International application number:
**PCT/EP2022/072049**

(87) International publication number:
**WO 2023/012322 (09.02.2023 Gazette 2023/06)**

(54) **LIPID-BASED COMPOSITION FOR ORAL ADMINISTRATION OF BRADYKININ B2-RECEPTOR ANTAGONISTS**

LIPIDBASIERTE ZUSAMMENSETZUNG ZUR ORALEN VERABREICHUNG VON BRADYKININ-B2-REZEPTOR-ANTAGONISTEN

COMPOSITION À BASE DE LIPIDES POUR L'ADMINISTRATION PAR VOIE ORALE D'ANTAGONISTES DES RÉCEPTEURS B2 DE LA BRADYKININE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA TN**

(30) Priority: **05.08.2021 EP 21189990**

(43) Date of publication of application:
**05.07.2023 Bulletin 2023/27**

(73) Proprietor: **Pharvaris GmbH**
**6300 Zug (CH)**

(72) Inventor: **GIBSON, Christoph**
**6300 Zug (CH)**

(74) Representative: **Walter, Kai U.**
**Paustian & Partner Patentanwälte mbB**
**Oberanger 32**
**80331 München (DE)**

(56) References cited:
**WO-A1-2019/101906 WO-A2-2008/055146**

- **ELNAGGAR Y S R ET AL: "Self-nanoemulsifying drug delivery systems of tamoxifen citrate: Design and optimization", INTERNATIONAL JOURNAL OF PHARMACEUTICS, ELSEVIER, NL, vol. 380, no. 1-2, 1 October 2009 (2009-10-01), pages 133-141, XP026715210, ISSN: 0378-5173, DOI: 10.1016/J.IJPHARM.2009.07.015 [retrieved on 2009-07-25]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 203 919 B1

## Description

BACKGROUND OF THE INVENTION

The present invention relates to pharmaceutical compositions comprising a bradykinin B2 receptor antagonist having a chemical structure according to Formula 1, to methods of preparing such compositions, and to their uses as medicaments in the treatment of subjects that may benefit from a bradykinin B2 receptor antagonist.

WO 2008/055146 discloses formulations of inhibitors of phospholipase enzymes, such as cytosolic $PLA_2$, compositions containing the same and processes for manufacture thereof.

Bradykinin (BK) is a peptide hormone that participates in inflammatory processes by activation of endothelial cells leading to vasodilation, increased vascular permeability, production of nitric oxide, and mobilization of arachidonic acid. BK also stimulates sensory nerve endings causing a burning dysaesthesia. Thus, the classical parameters of inflammation (e.g., redness, heat, swelling and pain) can all result from BK formation. BK is a short-lived component of the kallikrein-kinin system. The concentration of circulating BK is maintained at a low level under normal physiological conditions and may be rapidly increased under pathological situations by the enzymatic degradation of the circulating glycoprotein precursors called kininogens. The two most potent kininogen-metabolising enzymes are the trypsin-like serine proteases plasma kallikrein and tissue kallikrein. The precursors of these enzymes are normally present in all tissues and are ready to be activated by physiological or pathophysiological processes. The BK B2 receptor is constitutively expressed in most cell and tissue types and mediates most of the known effects of BK when this is produced in plasma or tissues. A large number of in vivo studies have shown that agents that blockade the BK B2 receptor provide therapeutic benefits in pathological conditions such as asthma, allergic rhinitis, pancreatitis, osteoarthritis, traumatic brain injury, Alzheimer's disease, and angioedema.

Numerous peptide and non-peptide antagonists of BK B2 receptor have been described in the prior art. Quinoline derivatives having activity as BK B2 receptor antagonists are, for example, disclosed in WO 2014/159637, WO 2010/031589, WO 2008/116620, WO 2006/40004, WO 03/103671, WO 03/87090, WO 00/23439, WO 00/50418, WO 99/64039, WO 97/41104, WO 97/28153, WO 97/07115, WO 96/13485, EP 0 795 547, EP 0 796 848, EP 0 867 432, and EP 1 213 289. However, these compounds showed a number of deficiencies hampering their utility as a drug, including low metabolic stability, low bioavailability, formation of glutathione adducts and bioactivation (toxicity) as disclosed in WO 2014/159637.

More recently, compounds of Formula 1 were proposed as novel, biologically active and well tolerated BK B2 receptor antagonists (see e.g. WO 2019/101906). While these compounds exhibit attractive pharmacological properties, they also show rather challenging physical or physicochemical properties, including very poor aqueous solubility in physiological media. Thus, there is a need for formulation designs and pharmaceutical compositions that overcome the difficulties resulting from these challenging compound properties such as to enable effective oral delivery and achieve significant systemic exposure and bioavailability in human subjects. There is also a need for formulations or pharmaceutical compositions incorporating compounds of Formula 1 such as to achieve a rapid onset of action by fast oral absorption (i.e. rapid absorption after oral administration) into the systemic circulation for the purpose of providing an effective treatment for acute symptoms or conditions associated with BK, which represents a particular challenge for such poorly soluble compound. A further need is the provision of formulations or pharmaceutical compositions incorporating compounds of Formula 1 that are stable in their performance and that can be manufactured by established pharmaceutical manufacturing technologies.

Compounds of Formula 1 have a very low water solubility, such as to make it extremely challenging to develop an oral formulation that would result in a sufficient rate and extent of bioavailability and efficacious plasma levels, in particular in a sufficiently rapid systemic absorption after oral administration that would allow effective non-invasive treatment of acute symptoms and conditions associated with BK.

An objective of the present invention is to address any one or more of these needs. A further objective is to overcome the deficiencies, gaps and limitations of the prior art with respect to the oral delivery of BK B2 receptor antagonists, such as compounds having a chemical structure according to Formula 1. Further objectives will become apparent on the basis of the following description, the Examples and the patent claims.

SUMMARY OF THE INVENTION

In one aspect, the invention relates to a liquid pharmaceutical composition for oral administration comprising a bradykinin (BK) B2 receptor antagonist having a chemical structure according to Formula 1, or a stereoisomer, salt or solvate thereof:

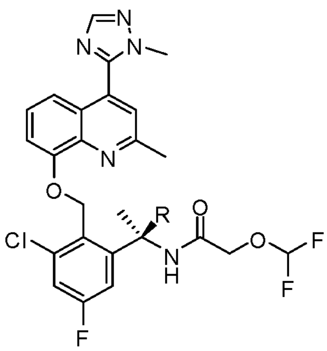

(Formula 1)

wherein R is deuterium or hydrogen; the composition is further characterised in that the BK B2 receptor antagonist is dissolved in a liquid vehicle comprising propylene glycol monocaprylate, polyoxyl castor oil, and propylene glycol. In particular, the BK B2 receptor antagonist may be (S)-N-(1-deutero-1-(3-chloro-5-fluoro-2-((2-methyl-4-(1-methyl-1H-1,2,4-triazol-5-yl)quinolin-8-yloxy)methyl)phenyl)ethyl)-2-(difluoromethoxy)acetamide, the propylene glycol monocaprylate may be a propylene glycol monocaprylate type II, and the polyoxyl castor oil may be a polyoxyl 40 hydrogenated castor oil.

In a further aspect, the invention relates to capsules, such as soft capsules or softgels, comprising such liquid pharmaceutical composition.

In yet a further aspect, the present invention relates to uses of such capsules or of liquid pharmaceutical compositions according to the invention, in particular in therapy. In general, the liquid pharmaceutical compositions or the capsules described herein may be used in the treament of diseases or conditions responsive to bradykinin B2 receptor modulation. For example, they are advantageous for the treatment of edema, such as hereditary angioedema.

DESCRIPTION OF THE DRAWINGS

Figure 1 shows individual API concentrations in the plasma of monkeys versus time in linear and semi-logarithmic scales. Figures 1A and 1B show the API concentration in plasma of 3 monkeys administered with the API in an aqueous carrier comprising methylcellulose (1 wt.%). Figures 1C and 1D show the API concentration in plasma of 3 monkeys administered with the API in a formulation according to the invention.

Figure 2 shows means and standard deviations of API concentration in plasma for human subjects administered with 1, 2, 4.5, 12, and 22 mg doses formulated according to the invention.

Figure 3 shows means and standard deviations of API concentration in plasma for human subjects administered with a 22 mg dose in the fasted state (hollow circles) or after high caloric/high fat (HCHF) breakfast (filled circles).

DETAILED DESCRIPTION OF THE INVENTION

The invention provides a liquid pharmaceutical composition for oral administration comprising a bradykinin (BK) B2 receptor antagonist having a chemical structure according to Formula 1, or a salt or solvate thereof:

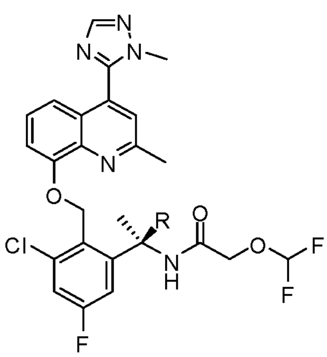

(Formula 1)

wherein R is deuterium or hydrogen; the composition being further characterised in that the BK B2 receptor antagonist is dissolved in a liquid vehicle comprising propylene glycol monocaprylate, polyoxyl castor oil, and propylene glycol.

It has been surprisingly found by the inventors that such a composition substantially enhances oral delivery of the BK B2 receptor antagonist in that it allows its incorporation in dissolved form, without susceptibility to rapid crystallisation upon dilution with aqueous media, and leads to unexpectedly rapid absorption of the compound into the bloodstream of a subject, which is particularly remarkable in view of its physical properties, in particular its large molecular size, the absence of a readily ionisable chemical group, and its low aqueous solubility.

In Formula 1, R may be selected from hydrogen and deuterium. In one preferred embodiment, or group of embodiments, R is deuterium:

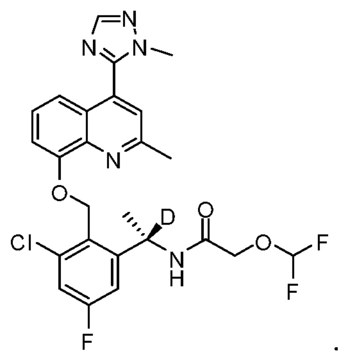

This compound which may also be referred to as (S)-N-(1-deutero-1-(3-chloro-5-fluoro-2-((2-methyl-4-(1-methyl-1H-1,2,4-triazol-5-yl)quinolin-8-yloxy)methyl)phenyl)ethyl)-2-(difluoromethoxy)acetamide (CAS 2340111-58-0), or alternatively as acetamide, N-[(1S)-1-[3-chloro-5-fluoro-2-[[[2-methyl-4-(1-methyl-1H-1,2,4-triazol-5-yl)-8-quinolinyl]oxy]methyl]phenyl]ethyl-1-d]-2-(difluoromethoxy)-, is a particularly advantageous example of a compound according to Formula 1 in the context of the invention. It should be understood that this preference also applies in combination with all other optional features or preferences disclosed in this description below, whether specifically mentioned or not.

Alternatively, in Formula 1, R may be hydrogen:

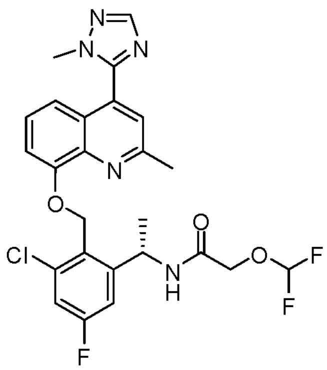

which may also be referred to as (S)-N-(1-(3-chloro-5-fluoro-2-((2-methyl-4-(1-methyl-1H-1,2,4-triazol-5-yl)quinolin-8-yloxy)methyl)phenyl)ethyl)-2-(difluoromethoxy)acetamide.

The compound of Formula 1 may be present in an essentially non-ionised form, or in ionised form, i.e. in the form of a salt Moreover, it may optionally be in the form of a solvate. For example, the compound may be a hydrate, such as (S)-N-(1-deutero-1-(3-chloro-5-fluoro-2-((2-methyl-4-(1-methyl-1H-1,2,4-triazol-5-yl)quinolin-8-yloxy)methyl)phenyl)ethyl)-2-(difluoromethoxy)acetamide monohydrate.

In one of the preferred embodiments, the state of the compound in the liquid composition is substantially non-ionised, such as entirely non-ionised. Moreover, the compound of Formula 1 as it is present in the liquid pharmaceutical composition described herein would normally not be in the form of a salt or solvate, or in any solid form. However, for the avoidance of doubt, the compound may be in solid form, or in the form of a salt and/or a solvate, when it is combined with the other components to form the composition of the invention. For example, a hydrate of the compound, such as the monohydrate, optionally in crystalline form, may be used for preparing the liquid pharmaceutical composition. It is believed, however, that upon being converted into the fully dissolved state, i.e. the form in which the compound is present in the liquid pharmaceutical composition, the compound is no longer in the form of a crystalline material or hydrate.

In one of the preferred embodiments, the BK B2 receptor antagonist of Formula 1 is the only active pharmaceutical ingredient (API) in the liquid pharmaceutical composition of the invention. This should also be understood as a general preference in the context of the present invention. Alternatively, the composition may comprise one or more further active ingredient(s).

A pharmaceutical composition, in the context of the present invention, should be understood as a composition that is technically suitable for being administered as a medicament to a subject, such as a human patient It is composed, formulated and processed in compliance with general pharmaceutical standards, as may be defined for example in the official pharmacopeias or guidances issued by the regulatory agencies such as the FDA and the EMA. In one of the preferred embodiments, the composition is adapted for oral administration, which implies, for example, that the excipients used, including their grades and their amounts, are safe and acceptable for oral use, in particular for oral administration to a human subject

The term "liquid", as used herein, refers to the liquid state of a material under normal conditions, i.e. at room temperature and under normal atmospheric pressure. An example of a more precisely defined set of normal conditions are the normal temperature and pressure (abbreviated as NTP) as defined by the National Institute of Standards and Technology (NIST) of the United States, which uses a temperature of 20 °C (293.15 K, 68 °F) and an absolute pressure of 1 atm (14.696 psi, 101.325 kPa).

A liquid vehicle, as used in the context of the invention, is a pharmaceutically acceptable liquid excipient or excipient mixture in which the at least one active pharmaceutical ingredient, such as the compound according to Formula 1, is incorporated. Formally, an API would not be considered as being part of the liquid vehicle, even if dissolved therein. Neither would any suspended solid excipient(s) be considered part of the vehicle. Thus, the weight of the liquid vehicle excludes the weight of the API(s) incorporated therein, and also the weight of materials suspended therein, if any are present. A liquid vehicle may also be referred to as a carrier.

For the avoidance of doubt, it is not necessary that a liquid vehicle including all its constituents is separately provided and then combined with the at least one API to form the liquid pharmaceutical composition of the invention. Rather, it is also possible to dissolve the at least one API in one of the liquid constituents of the liquid vehicle and to subsequently add the remaining constituents, or to combine all constituents of the liquid pharmaceutical composition simultaneously. Also, for the avoidance of doubt, it is not required that all constituents of the liquid vehicle are per se (individually) liquids under normal conditions, provided that they form a liquid phase (i.e. dissolve) when combined.

The term "pharmaceutically acceptable" as used herein means approved or approvable by a regulatory agency of the Federal or a state government or the corresponding agency in countries other than the United States, or that is listed in the U.S. Pharmacopoeia or other generally recognized pharmacopoeia for use in animals, and more particularly, in humans.In particular, pharmaceutically acceptable means that the pharmaceutically active compound(s) and other ingredients used in the pharmaceutical compositions and methods described herein are suitable for use in contact with the tissues of humans and lower animals without undue toxicity, irritation, allergic response, and the like, commensurate with a reasonable benefit/risk ratio.

As mentioned, the liquid pharmaceutical composition comprises the BK B2 receptor antagonist which is dissolved in a liquid vehicle as defined herein. In this context, the expression "dissolved" refers to the state of being dissolved, i.e. to the presence of the compound in a fully dissolved state. This implies that the compound is molecularly dispersed in the liquid vehicle, rather than being incorporated in the form of suspended particles. Hence, the BK B2 receptor antagonist, which may in this context also be referred to as the active ingredient, API or drug substance, is present in the liquid pharmaceutical composition in a non-solid form.

As used herein, the expression "propylene glycol monocaprylate" should be understood in the context of pharmaceutics, rather than strict chemical nomenclature. In the context of pharmaceutics, propylene glycol monocaprylate refers to an excipient that complies with a commonly accepted compendium monograph relating to propylene glycol monocaprylate. This includes, for example, the monographs "Propylene Glycol Monocaprylate Type I" and "Propylene Glycol Monocaprylate Type II" of the United States Pharmacopeia and The National Formulary (USP/NF), e.g. in its version USP-NF 2021, and/or to related monographs in other pharmacopoeias such as the European Pharmacopeia (Ph.Eur.).

Generally speaking, a material or excipient designated as propylene glycol monocaprylate comprises a mixture of several chemical species. It may be described as a mixture of the propylene glycol monoesters and diesters of fatty acids composed predominately of caprylic acid. The monoester and diester content may differ between the type of propylene glycol monocaprylate: According to the USP-NF, an excipient representing a propylene glycol monocaprylate type I contains from 55.0 to 80.0 percent monoesters and from 20 to 45 percent diesters, whereas a material representing a propylene glycol monocaprylate type II contains at least 90.0 percent monoesters and not more than 10.0 percent diesters. With respect to the fatty acid residues in either type I or type II, at least 90.0 percent of the fatty acid esters are caprylates (or octanoates), and not more than 3.0 percent - individually for each of these residues - are caprates (or decanoates), laurates (or dodecanoates) and myristates (or tetradecanoates), respectively. Neither type I nor type II propylene glycol monocaprylate contains more than 1.0 percent palmitates (or hexadecanoates). Non-limiting examples

of currently available commercial grades of propylene glycol monocaprylate type I include Capryol® PGMC (Gattefossé) and Capmul® PG-8-70 NF (Abitec), and commercially available versions of propylene glycol monocaprylate type II include Capmul® PG-8 NF (Abitec) and Capryol® 90 (Gattefossé).

In one of the preferred embodiments, the propylene glycol monocaprylate comprised in the liquid vehicle comprised in the liquid pharmaceutical composition of the invention is a propylene glycol monocaprylate type II, such as propylene glycol monocaprylate type II (USP/NF). In another preferred embodiment, the propylene glycol monocaprylate type II is the only type of propylene glycol monocaprylate incorporated in the liquid vehicle. Alternatively, a mixture of propylene glycol monocaprylates may also be used.

In a further embodiment, a propylene glycol monocaprylate is selected which exhibits a hydrophilic-lipophilic-balance (HLB value) in the range of about 5 to 6.

Similarly, the term "polyoxyl castor oil", according to the technical context of the present invention, should be interpreted to mean an excipient or material that conforms to the commonly accepted pharmaceutical standards for any polyoxyl castor oil as established in the respective monographs of the relevant compendia, such as the pharmacopoeias referred to above. Thus, the term refers to pharmaceutical grades of polyoxyethylene castor oil derivatives. Polyoxyl castor oils are mixtures of different chemical species and typically produced by reacting ethylene oxide with castor oil or hydrogenated castor oil.

One type of polyoxyl castor oil is a material that complies with the monograph, "Polyoxyl 40 Hydrogenated Castor Oil" (USP, current edition), which essentially corresponds to the monograph, "Macrogolglycerol Hydroxystearate" (Ph. Eur., current edition), also referred to as PEG-40 hydrogenated castor oil. Polyoxyl 40 hydrogenated castor oil typically occurs as a white to yellowish, semisolid paste at room temperature that liquefies above about 30 °C. The main constituent of this excipient is glycerol polyethylene glycol hydroxystearate, and it further comprises fatty acid glycerol polyglycol esters, polyethylene glycols and glycerol ethoxylate. According to the European Pharmacopeia, it contains mainly the reaction product of trihydroxystearyl glycerol ethoxylated with 7 to 60 molecules of ethylene oxide (nominal value), with small amounts of macrogol hydroxystearate and of the corresponding free glycols. Examples of commercially available grades of polyoxyl 40 hydrogenated castor oil include Kolliphor® RH40 (BASF), previously sold under the name Cremophor® RH40, and Croduret (Croda).

Another type of polyoxyl castor oil conforms to the monograph "Polyoxyl 35 Castor Oil" (USP, current edition), which corresponds to the monograph "Macrogolglycerol Ricinoleate" (Ph. Eur., current edition), and is also referred to as PEG-35 castor oil. It contains mainly ricinoleyl glycerol ethoxylated with 30-50 molecules of ethylene oxide (nominal value), with small amounts of macrogol ricinoleate and of the corresponding free glycols. It results from the reaction of castor oil with ethyleneoxide. Examples of commercially available grades of polyoxyl 35 castor oil include Kolliphor® EL (BASF), previously marketed as Cremophor® EL.

In one of the preferred embodiments, the polyoxyl castor oil comprised in the liquid vehicle of the liquid pharmaceutical composition of the invention is polyoxyl 40 hydrogenated castor oil, such as polyoxyl 40 hydrogenated castor oil (USP/NF). In another preferred embodiment, polyoxyl 40 hydrogenated castor oil is the only type of polyoxyl castor oil incorporated in the liquid vehicle. Alternatively, a further type of polyoxyl castor oil may also be present.

According to a further preferred embodiment, the polyoxyl castor oil comprised in the liquid vehicle is polyoxyl 40 hydrogenated castor oil and the propylene glycol monocaprylate is a propylene glycol monocaprylate type II, and other types of polyoxyl castor oil or propylene glycol monocaprylate are absent. In other words, the liquid pharmaceutical composition may comprise the compound of Formula 1 dissolved in a liquid vehicle comprising polyoxyl 40 hydrogenated castor oil, propylene glycol monocaprylate type II and propylene glycol, as defined herein, and no other propylene glycol monocaprylate or polyoxyl castor oil.

According to a further preferred embodiment, the liquid vehicle, and therefore also the pharmaceutical composition of the invention, can further comprise water. The water may be added deliberately as part of the constituents of the liquid vehicle, or it may become part of the liquid vehicle as a result of the water content of the raw materials or intermediate products used for the preparation of the liquid pharmaceutical composition or its further processing, e.g. by encapsulating it within a soft gelatine capsule. For example, if the compound of Formula 1 used for preparing the liquid pharmaceutical composition is provided in the form of a crystalline hydrate, the crystal water of the hydrate would become part of the liquid vehicle. Moreover, if the liquid pharmaceutical composition is combined with a wet gelatine mass as typically used in the preparation of soft gelatine capsules, some of the water of the gelatine mass may migrate into the liquid pharmaceutical composition and form part of the liquid vehicle.

In a further preferred embodiment of the liquid pharmaceutical composition of the invention, the bradykinin B2 receptor antagonist is a compound according to Formula 1 wherein R is deuterium, and it is dissolved in a liquid vehicle comprising polyoxyl 40 hydrogenated castor oil, propylene glycol monocaprylate type II, propylene glycol and water.

In general, the liquid vehicle may optionally comprise one or more further excipients. In one embodiment, it may comprise a further solvent and/or a further surfactant In this context, a further solvent means a solvent further to propylene glycol, which is in any case present according to the invention. Preferably, the solvent is an organic solvent, such as a water-miscible organic solvent, such as a pharmaceutically acceptable water-miscible organic solvent, such

as glycerol or ethanol. In one embodiment, the liquid vehicle comprises ethanol.

In one embodiment, a further surfactant may be used, such as an additional pharmaceutically acceptable surfactant. In this context, a "further surfactant" means a surfactant in addition to the propylene glycol monocaprylate and the polyoxyl castor oil, either of which may be considered as a surfactant, even though a few other functional labels may also be used for these excipients. In a preferred embodiment, the liquid vehicle can comprise caprylocaproyl polyoxyl-8 glycerides as a further surfactant, also referred to as caprylocaproyl macrogol-8 glycerides. An example of a commercially available excipient representing caprylocaproyl polyoxyl-8 glycerides is the product Labrasol® ALF (Gattefossé).

Moreover, one or more further excipients may be incorporated in the liquid pharmaceutical composition, optionally as part of the liquid vehicle (e.g., in case of incorporation of one or more liquid excipients), and may be selected from stabilisers, antioxidants, preservatives, pH-modifying agents, taste-modifying agents, colouring agents, and viscosity-modifying agents. Mixtures or combination of two or more of the afore-mentioned further excipients may also be used.

In one embodiment, the liquid pharmaceutical composition is adapted for multiple dosing, such as a liquid presented in a multiple dose container, and further characterised in that it comprises at least one taste-modifying agent and is free of any preservatives. In fact, it is one of the advantages of the present invention that it does not require the addition of a preservative even when presented as a multi-dose liquid formulation. In this context, the term "preservative" should be understood as referring to an excipient whose only or primary function is that of delivering a safe and permanent antimicrobial function. An example of a preservative is an antimicrobial preservative, such as benzoic acid and salts thereof, sorbic acid and salts thereof, methyl paraben, propyl paraben, and the like.

As mentioned, the inventors have surprisingly found that a liquid pharmaceutical composition as described herein shows a remarkable performance, both *in vitro* and *in vivo.* It substantially enhances oral delivery of the BK B2 receptor antagonist in that it allows its incorporation in fully dissolved (non-solid) form, without susceptibility to rapid crystallisation upon dilution with aqueous media, such as water, acidified water or simulated gastric fluid. Without wishing to be bound by theory, the inventors believe that the liquid pharmaceutical compositions described herein behave as, or represent, so-called self-emulsifying or self-microemulsifying drug delivery systems (SEDDS or SMEDDS, respectively). These may be described as isotropic liquid mixtures that spontaneously form oil-in-water (o/w) emulsions or -microemulsions when diluted with an aqueous medium, typically even without the need of mechanical agitation. In the context of the present invention, SEDDS should be understood as a broader expression which encompasses SMEDDS. SMEDDS are characterized by a small average droplet size of < 800 nm, sometimes even in the range of 100 nm. In one of the preferred embodiments, the liquid pharmaceutical composition is in the form of a SMEDDS. This is a general preference and should be understood as being applicable also in combination with all other preferences described herein.

Even though SEDDS have been suggested as a potential formulation strategy for poorly soluble drug substances, not many drug products have actually been successfully developed as SEDDS and received market approval. Many SEDDS formulations fail in that they do not achieve a sufficient drug load (such that a single dose of the drug cannot be accommodated in e.g. one or two soft gelatine capsules), or do not incorporate the active ingredient in such a way that it remains dissolved in the (micro)emulsion that forms upon dilution with water or gastric fluid. In fact, even in the case of sufficient solubility of the active ingredient in the liquid carrier a frequent problem is that rapid precipitation of the active ingredient occurs, which is one of the reasons why SEDDS formulation strategies are often unsuccessful in practice. In addition, drug substances dissolved in SEDDS very often show poor stability and rapid chemical degradation leading to a short shelf-life that is unattractive or even unfeasible from the marketing and supply chain perspective.

Also in the case of the BK B2 receptor antagonist according to Formula 1, the inventors found that many liquid vehicle compositions that were expected to have self-emulsifying or self-microemulsifying properties are not compatible with this active ingredient in that they show rapid drug precipitation or liquid phase separation when the compound of Formula 1 is incorporated. However, the inventors have unexpectedly found one particular combination of excipients, namely of propylene glycol monocaprylate, polyoxyl castor oil and propylene glycol, that neither leads to precipitation of the compound nor to liquid phase separation within several hours after dilution with acidified water at room temperature immediately after production as demonstrated in the Examples. More importantly, and particularly noteworthy, the inventors found that this liquid vehicle provides excellent stability and allows for sufficient drug load. No precipitation of the BK B2 receptor antagonist nor liquid phase separation was observed after 6 months storage at elevated temperature, such as 40°C, as also illustrated by the Examples.

In addition to the preferred embodiments with respect to further constituents of the liquid vehicle or of the liquid pharmaceutical composition of the invention as described above, the inventors have also found that certain quantities of the respective excipients in the liquid vehicle seem particularly advantageous. In particular, the inventors have found that relatively high amounts of the propylene glycol monocaprylate are advantageous, such as about 40 wt.-% or higher, relative to the weight of the liquid vehicle, which is significantly above the amounts used in some known SMEDDS formulations of other drug substances. For example, in a further preferred embodiment, the amount of the propylene glycol monocaprylate in the liquid vehicle is about 40-60 wt.%, such as about 45-55 wt.%, such as about 48-52 wt%,

based on the weight of the liquid vehicle. In case more than one type of propylene glycol monocaprylate is used, the amount of about 40-60 wt.% should be understood as referring to the total amount of all propylene glycol monocaprylate in the liquid vehicle. In a related embodiment, the liquid vehicle comprises about 40-60 wt.%, such as about 45-55 wt.%, such as about 48-52 wt%, of propylene glycol monocaprylate type II.

In a further preferred embodiment, the amount of polyoxyl castor oil in the liquid composition is about 30-50 wt.%, such as about 35-45 wt.%, such as about 38-42 wt%, based on the weight of the liquid vehicle. Again, the range refers to the total amount of polyoxyl castor oil in the liquid vehicle in case that more than one type of polyoxyl castor oil is present in the vehicle. Moreover, it is also a preferred embodiment if the entire 30-50 wt% of polyoxyl castor oil represent polyoxyl 40 hydrogenated castor oil. In one embodiment, the liquid composition comprises about 40-60 wt.% such as about 45-55 wt%, such as about 48-52 wt%, of propylene glycol monocaprylate (preferably of type II) and 30-50 wt%, such as about 35-45 wt%, such as about 38-42 wt.%,of polyoxyl castor oil (preferably polyoxyl 40 hydrogenated castor oil).

With respect to the amount of propylene glycol in the liquid vehicle, it was found that relatively low amounts are advantageous, such as about 15 wt.-% or less, based on the total weight of the liquid vehicle, and preferably a range of about 2.5-15 wt% is advantageous. Also preferred are propylene glycol amounts in the range of about 2.5-11 wt.%, such as about 3.5-11 wt.%, or such as about 4.5-10 wt.%., again based on the total weight of the liquid vehicle

In a preferred embodiment, the liquid vehicle comprises propylene glycol monocaprylate (preferably of type II), polyoxyl castor oil (preferably polyoxyl 40 hydrogenated castor oil), propylene glycol in the amounts described above, and, optionally, further water as balance. For example, the liquid vehicle may comprise about 40-60 wt% of propylene glycol monocaprylate (preferably of type II), 30-50 wt.% polyoxyl castor oil (preferably polyoxyl 40 hydrogenated castor oil) and 2.5-15 wt.% of propylene glycol.

The liquid pharmaceutical composition described herein can also comprise water. The water may be present in an amount of up to 5 wt%. For example, water may be a constituent of the liquid vehicle, such as the liquid vehicle may comprise water at an amount of up to 5 wt%. In this context, it is noted that in case the liquid pharmaceutical composition is intended for encapsulation, e.g. in soft gelatine capsules as further described below, the content of certain constituents including propylene glycol and/or water may change during storage over time, partly because of the possibility that some of the encapsulated propylene glycol and/or water migrates into the capsule shell. For example, the liquid pharmaceutical composition may be manufactured such as to exhibit a propylene glycol content of about 10 wt.%, but after encapsulation into soft gelatine capsules and storage over several months or years the propylene glycol content in the liquid fill of the soft gelatine capsule may be less, such as about 8 or 9 wt.%, due to migration. Vice versa, the capsule wall is prepared such as to include propylene glycol, the migrated amount of propylene glycol may have increased the propylene glycol content of the capsule wall after storage.

In a yet further preferred embodiment of the liquid pharmaceutical composition, the content of propylene glycol monocaprylate (preferably of type II) is about 50 wt.%, the content of polyoxyl castor oil (preferably polyoxyl 40 hydrogenated castor oil) is about 40 wt.%, and the content of propylene glycol is about 10 wt.%, based on the combined weight of propylene glycol monocaprylate, polyoxyl castor oil, and propylene glycol in the liquid vehicle. As used herein, the term "about" refers to possible minor variations, e.g. to compensate for minor differences between various grade of an excipient, keeping in mind that some of these are themselves mixtures of different chemical species (e.g. in the case of propylene glycol monocaprylate or polyoxyl castor oil) or may contain variable amounts of certain impurities such as water (e.g. in the case of propylene glycol). For example, a relative deviation of up to 10% (as in 50$\pm$5 wt.%), such as 1%, 2%, 3%, 4%, 5%, 6%, 7%, 8%, or 9%, would normally be considered as a composition having essentially the same function.

According to another preferred embodiment of the liquid pharmaceutical composition, the liquid vehicle essentially consists of propylene glycol monocaprylate, polyoxyl castor oil, propylene glycol and not more than about 10 wt% other liquid components, based on the combined weight of all liquid constituents in the liquid vehicle. As mentioned above, the liquid vehicle may comprise a further liquid constituent, such as an optionally liquid surfactant, water or an organic solvent, such as ethanol, and according to this specific embodiment the total amount of these is restricted to about 10 wt% relative to the weight of the liquid vehicle.

With respect to the content of the BK B2 receptor antagonist of Formula 1 in the liquid pharmaceutical composition, this should be selected in view of the type of subject to be treated (e.g. paediatric human patient, adult human patent), the therapeutic indication and the presentation of the composition (i.e. whether presented e.g. as a liquid for oral administration, as a soft gelatine capsule, etc.). Preferably, the relative amount of the compound is in the range from about 1 mg to about 160 mg per g of the liquid composition. In this context, the amount of the BK B2 receptor antagonist of Formula 1 when expressed as a weight should be interpreted as referring to the amount of the non-ionised, non-solvated form of the active ingredient, or in other words the pharmacologically active part of the compound that is also declared as the dose. If, for example, a hydrate form of the compound is used to prepare the liquid pharmaceutical composition, the weight of the active ingredient excludes the water of hydration (which would be part of the liquid vehicle, as explained above). In a specific embodiment, the amount of the compound of Formula 1 is at least about 5 mg per g

of the liquid composition, corresponding to about 0.5 wt%. In another specific embodiment, said amount is at least about 10 mg per g of the liquid composition, corresponding to about 1 wt.%.

In a further preferred embodiment, the liquid pharmaceutical composition exhibits a content of the BK B2 receptor antagonist in the range from about 5 mg to about 100 mg per g of the liquid pharmaceutical composition, such as in the range from about 20 mg to about 70 mg per g. In a preferred embodiment, its content is in the range from about 5 mg to about 65 mg per g, such as in the range from about 5 mg to about 50 mg per g. It is a particular advantage of the liquid pharmaceutical composition of the invention that the liquid vehicle identified by the inventors and described herein is capable of accommodating such relatively large amounts of the active ingredient. In further preferred embodiments, the content of the BK B2 receptor antagonist is in the range from about 10 mg to about 65 mg per g, such as in the range from about 20 mg to about 65 mg per g. In specific embodiments, said content is about 20, 25, 30, 40 or 50 mg per g, corresponding to about 2, 2.5, 3, 4 or 5 wt%, respectively.

With respect to all amounts of the BK B2 receptor antagonist discussed above, it should be understood that these preferences also apply specifically to the BK B2 receptor antagonist according to Formula 1 wherein R is deuterium.

A further preferred liquid pharmaceutical composition can essentially consist of:
(a) based on the total weight of the liquid pharmaceutical composition, about 0.5 to 6.5 wt.%, such as 5 wt.%, of a compound according to Formula 1, or a stereoisomer, salt or solvate thereof, wherein R is deuterium; (b) based on the total weight of the liquid pharmaceutical composition, about 93.5 to 99.5 wt% of a liquid vehicle; wherein said liquid vehicle essentially consists of: (i) about 50 parts (in weight) of propylene glycol monocaprylate, such as propylene glycol monocaprylate type II; (ii) about 40 parts (in weight) of polyoxyl castor oil, such as 40 hydrogenated castor oil; (iii) about 5 to 10 parts (in weight) of propylene glycol; and (iv) up to about 5 parts (in weight) of water; and optionally (c) the balance being one or more further excipients dissolved or dispersed in the liquid vehicle. In a further specific embodiment, said liquid pharmaceutical composition comprises about 5 wt% of the compound of Formula 1 and no further excipients dissolved or dispersed in the liquid vehicle.

The liquid pharmaceutical composition of the invention may be used as such, i.e. as a medicament formulated and presented as a liquid for oral administration, or it may be further processed, e.g. by incorporating it into a solid single unit dosage form such as a capsule. For a presentation as a liquid dosage form, it may be filled into a suitable primary packaging unit or container such as to comprise either a single dose or multiple doses of the active ingredient. As used herein, a primary packaging unit is a packaging means or combination of packaging means that holds the drug formulation such as to be directly in contact with it. An example of a combination of packaging means which together form a primary packaging unit is a bottle together with a screw-on lid.

A single dose may be accommodated, for example, in a glass or plastic bottle, vial or ampoule. Alternatively, a sachet or stick pack may be used. As primary packaging unit for holding multiple doses of the liquid composition, glass or plastic bottles are suitable. In order to facilitate the withdrawal of a measured amount of the composition, a dosing or dispensing aid may be used, which may be part of the primary packaging unit or separate. According to one such embodiment, the invention provides a primary packaging unit comprising the liquid composition as described above and a dosing or dispensing aid. In a further embodiment, the dosing or dispensing aid is a dosing pump. Optionally, the dosing pump is directly connectable to the primary packaging means; for example, the primary packaging container may be a glass or plastic bottle provided with a lid that may be screwed off and replaced by a screw-on dosing pump.

In a further preferred embodiment, the liquid pharmaceutical composition is presented as a single dose unit in the form of a capsule that is filled with the liquid pharmaceutical composition. Accordingly, one further aspect of the invention is a capsule for oral administration comprising the liquid pharmaceutical composition as described above. Optionally, the capsule may be a hard capsule, in particular a hard gelatine capsule.

In one of the preferred embodiments, the capsule is a soft capsule, also referred to as a soft gelatine capsule or softgel. More specifically, the soft capsule preferably comprises a capsule wall comprising gelatine, water, and at least one plasticiser. In this context, a capsule wall means the shell of a capsule which surrounds, or encapsulates, the liquid fill material, which is in the present case the liquid pharmaceutical composition as disclosed herein. Also in the context of soft gelatine capsules, a "plasticiser" is a pharmaceutical excipient that is used to make the capsule wall more elastic and pliable and to minimise its brittleness and the risk of cracking. To some extent, water also has a plasticising effect on the gelatine capsule wall material; in the context of the present invention, however, the term "plasticiser" should be understood as excluding water.

The main component of the soft gelatine capsule wall is typically the gelatine itself. Gelatine is a generic term for a mixture of purified protein fractions obtained either by partial acid hydrolysis (type A gelatine) or by partial alkaline hydrolysis (type B gelatine) of animal collagen obtained from cattle and pig bone, cattle skin (hide), pigskin, and fish skin. Gelatine may also be a mixture of both types. The protein fractions consist almost entirely of amino acids joined together by amide linkages to form linear polymers, varying in molecular weight from 20 000 to 200 000.

The mechanical strength of a gelatine material may be characterised by the Bloom number, also referred to as Bloom value, which is determined by the Bloom test, which expresses the weight in grams needed by a standardised plunger to depress the surface of a gelatine gel sample by 4 mm without breaking it. Typically, the gelatine gel sample

is prepared as a 6.67% gelatine solution which is solidified at about 10 °C for several hours. In general, a higher Bloom number is found for gelatine materials with a high average molecular weight, and vice versa. If a mixture of different types of gelatine is used, the Bloom value - in the context of the present invention - should be interpreted as relating to the mixture.

The inventors found that, in principle, both types of gelatine may be used for making the soft capsules according to the invention, i.e. type A and type B. Moreover, gelatines of different gel strengths may also be used as longer as they are adequately plasticised. In one embodiment, the gelatine of the capsule wall has a Bloom value in the range from about 100 to about 250. In another preferred embodiment, the Bloom value (or Bloom number) of the gelatine is in the range from about 130 to about 220, and in further embodiments, the Bloom value is about 150±20 or about 200±20, respectively. In some further specific embodiments, the gelatine is a type A gelatine with a Bloom value of about 195, or it is a type B gelatine with a Bloom value of about 150, respectively.

With respect to the plasticiser, this may, for example, be selected from glycerol, propylene glycol, polyethylene glycol, sorbitol, sorbitan, maltitol, corn syrup, citric acid esters such as triethyl citrate, or combinations of any of these. In one preferred embodiment, the soft gelatine capsule comprises a capsule wall comprising gelatine, water, and at least one plasticiser selected from propylene glycol, glycerol, sorbitol, sorbitan, sorbitol-based plasticiser mixtures, or any combinations thereof.

If sorbitan is used, it is preferred that sorbitol is also used. For example, a mixture of sorbitol and sorbitan (currently marketed e.g. as Sorbitol Special® by ISP) may be used. Such mixtures of sorbitol with one or more further excipients to modify the plasticiser properties, also referred to as sorbitol-based plasticiser mixture, typically also have the advantage that they comprise sorbitol in an essentially non-crystallisable form. In this context, the expression "non-crystallisable sorbitol" is also used to encompass sorbitol-based plasticiser mixtures such as sorbitol-sorbitan combinations, e.g. Sorbitol Special®, in which the sorbitol is rendered non-crystallisable by the addition of sorbitan and/or another additive. A further preferred sorbitol-based plasticiser mixture is Polysorb® 85/70/00 marketed by Roquette, which is described as liquid, partially dehydrated sorbitol. More precisely, the product is a mixture of sorbitol (20-40%), 1,4-anhydro-D-glucitol (20-30%) and hydrogenated corn syrup (20-25%). Such products are also described in the USP monograph, "Sorbitol Sorbitan Solution" (formerly "Anhydrized liquid sorbitol"), or in the Ph.Eur. monograph, "Sorbitol, liquid, partially dehydrated".

In a further preferred embodiment, the capsule wall is plasticised at least with glycerol. Also preferred is a capsule wall composition which comprises glycerol and at least one further plasticiser. In one preferred embodiment, the second plasticiser is selected from non-crystallisable sorbitol (or sorbitol-sorbitan or partially dehydrated liquid sorbitol) and propylene glycol.

The amount of the plasticiser, or in case more than one plasticiser is used, the total plasticiser amount in the capsule wall composition before encapsulation and drying of the capsules (i.e. the amount in the wet gel composition used for the encapsulation process) should preferably be selected in the range of about 15-35 wt%, relative to the total weight of the wet capsule wall composition. Once again, in the context of the present invention, water is excluded from the plasticiser amount In another preferred embodiment, the plasticiser amount in the capsule wall composition is in the range of about 15-30 wt.%, and in particular in the range of about 18-28 wt%.

The amount of plasticiser may also be expressed as a ratio of the (total) gelatine amount to the (total) plasticiser amount in the capsule wall. The advantage of referring to this ratio is that it is relatively independent of the water content; it does not differ dramatically between the wet gelatine mass and the dried soft gelatine capsule wall after manufacture, although it may change over time due to the possible migration of plasticiser out of the capsule wall into the capsule fill, or vice versa. In some preferred embodiments, the ratio is selected in the range of about 1.0 to 3.0, or in the range of about 1.3 to 2.8, or about 1.3 to 2.5, respectively.

If the soft capsule wall comprises a second plasticiser in addition to glycerol, which is preferably propylene glycol or sorbitol (including sorbitol-sorbitan mixtures or partially dehydrated liquid sorbitol), the weight ratio of glycerol to the second plasticiser may be selected as generally known in the art For example, the ratio may be in the range of about 0.1-10, or in the range of about 0.2-5. In one of the preferred embodiments, the ratio is in the range of about 0.5-2.

It was found by the inventors that when observing the preferences with respect to the composition of the soft gelatine capsule wall, the liquid composition could be successfully encapsulated, and that capsule brittleness and pellicle formation can largely be avoided.

The amount of water in the soft capsule wall, or initially in the wet gel mass provided for preparing the capsules, may be selected in the typical ranges as required to ensure processability. For example, the wet gelatine mass may have an initial water content of about 20-60 wt.%. In some embodiments, the water content is selected in the range of about 25-45 wt%. It is noted that the water content should be calculated such as to include the water introduced by other excipients, such as glycerol (e.g. if glycerol 85% is used) or sorbitol-sorbitan solution. The weight ratio of the (total) gelatine to the (total) water may also be selected as commonly used in softgel manufacture, i.e. in the range of about 0.5-2. Optionally, the capsule wall may comprise one or more further ingredients, such as one or more excipients selected from colouring agents, pigments, opacifiers, flavours, and lubricants. Typically, these excipients may be incorporated at

relatively low amounts in order for them to perform their respective functions. For example, titanium dioxide may be used as an opacifier, typically at an amount of up to 3 wt.%. Similarly, if a coloured capsule is desired, colourants such as one or more iron oxides may be added, typically also in low amounts, such as up to about 5 wt.%. The capsule wall may also comprise small amounts of processing aids, such as lubricants. An example of a potentially suitable lubricant is a neutral fatty oil (liquid trigylcerides). Optionally, a surfactant such as lecithin may be added to the oil.

According to a further aspect, the invention relates to the use of the liquid pharmaceutical composition as described herein for the manufacture of a capsule, in particular a soft capsule as also described above. Expressed in terms of a process, the invention provides a method for preparing a soft capsule using the liquid pharmaceutical composition as described herein. The method may be characterised by the following steps:

(a) providing the liquid composition as described herein;

(b) providing a wet capsule wall material comprising gelatine, water, a first plasticiser which is glycerol and a second plasticiser which is selected from sorbitol and propylene glycol;

(c) encapsulating the liquid composition within the wet capsule material such as to form a capsule;

(d) drying the capsule formed in step (c); and optionally

(e) storing the dried capsule.

With respect to the process parameters, the method may be carried out using standard equipment and settings. With respect to the liquid pharmaceutical composition provided in step (a), it is emphasised that the same optional features and preferences apply as have been described in the context of that aspect of the invention. For example, it is also one of the preferred embodiments of the method for preparing a soft capsule wherein, in step (a), a liquid composition is provided which essentially consists of:

- based on the total weight of the liquid composition, about 0.5 to 6.5 wt.% - such as 5 wt% - of a compound according to Formula 1, or a stereoisomer, salt or solvate thereof, wherein R is deuterium;
- based on the total weight of the liquid composition, about 93.5 to 99.5 wt% of a liquid vehicle, said vehicle essentially consisting of:

  (i) about 50 parts (in weight) of propylene glycol monocaprylate, such as propylene glycol monocaprylate type II;
  (ii) about 40 parts (in weight) of castor oil, such as 40 hydrogenated castor oil;
  (iii) about 5 to 10 parts (in weight) of propylene glycol; and optionally
  (iv) up to about 5 parts (in weight) of water;

  and optionally
- the balance being one or more further excipients dissolved or dispersed in the liquid vehicle.

In a further preferred embodiment, the invention provides a soft capsule obtainable by the method characterised by steps (a) to (e). In view of the changes that a soft capsule may undergo during its shelf life, e.g. due to the migration of a plasticiser from the capsule wall into the fill liquid or the migration of an ingredient from the liquid fill into the capsule wall, thus changing the quantitative composition of both the liquid fill and of the capsule wall over time, it appears entirely appropriate to define the soft capsule in terms of its manufacturing process and its starting (or intermediate) materials.

According to a further aspect of the invention, the liquid pharmaceutical composition, or the capsule comprising such composition, may be used in the acute or chronic treatment of a subject suffering from any disease or condition responsive to bradykinin B2 receptor modulation. Examples of diseases or conditions responsive to BK B2 receptor modulation, include a disease or condition such as a skin disorder; eye disease; ear disease; mouth, throat and respiratory disease; gastrointestinal disease; liver, gallbladder and pancreatic disease; urinary tract and kidney disease; disease of male genital organs and female genital organs; disease of the hormone system; metabolic disease; cardiovascular disease; blood disease; lymphatic disease; disorder of the central nervous system; brain disorder; musculoskeletal system disease; allergy disorder; pain; infectious disease; inflammatory disorder; injury; immunology disorder; cancer; hereditary disease; and edema. Expressed in other words, one aspect of the invention relates to a method of treating a subject suffering from any disease or condition responsive to bradykinin B2 receptor modulation, wherein the method comprises the administration of a composition or capsule as described above. Similarly, the invention provides the use of a composition or capsule described herein in the manufacture of a medicament for the treatment of any disease or condition responsive to bradykinin B2 receptor modulation, such as acute or chronic treatment of a disease or condition

responsive to BK B2 receptor modulation.

As used herein, the expressions "treatment", "treating" and the like should be interpreted to include any type of prophylactic or therapeutic treatment. As such, it encompasses the prevention, management or therapy of a disease or its reoccurrence, or of any symptoms associated with such disease or condition. Moreover, in the context of the invention, "acute" includes any non-chronic administration regimen, for example a single administration of an effective single dose of a composition or capsule described herein, as well as a sporadic or regular dosing regimen over a relatively short period of time, such as up to four weeks, or up to two weeks. In one of the preferred embodiments, the liquid composition or the capsule provided by the invention is used for the acute treatment of a disease or condition responsive to bradykinin B2 receptor modulation.

The following diseases or conditions may be considered as responsive, or at least potentially responsive, to bradykinin B2 receptor modulation:

Skin disorders, including, without limitation, disorders such as skin aging, skin efflorescences including pressure sores, decubital ulcers, irritated, sensitive and dysaesthetic skin, erythema, rash, skin edema, psoriasis, eczema, Netherton syndrome, lichen, bacterial, viral, fungal and parasites induced skin infections including furuncle, abscess, phlegmon, erysipelas, folliculitis and impetigo, lice, scabies and herpes simplex, acne, exanthema, dermatitis including atopic dermatitis, allergic contact dermatitis, neurodermatitis, radiation damage, sunburn, pruritus, itching, cholestatic pruritus, chronic pruritus, chronic prurigo, prurigo nodularis, urticaria, chronic spontaneous urticaria, chronic inducible urticaria, cold urticaria, cryopyrin-associated periodic syndromes (CAPS), familial cold auto-inflammatory syndrome (FCAS), FXII-associated cold autoinflammatory syndrome (FACAs), psoriasis, mycosis, tissue ulceration, epidermolysis bullosa, wounds including abnormal wound healing, burns, frostbite, skin inflammation and edema caused by venoms, alopecia, hair squama, corn, wart and panaris.

Eye diseases, including, without limitation, inflammatory disorders such as scleritis, conjunctivitis, chemosis, iritis, iridocyclitis, uveitis, chorioretinitis, as well as disorders such as retinochoroidal circulatory disorders, bacterial eye infections, unspecific conjunctivitis and eye irritations, retinopathy of prematurity, proliferative vitreoretinopathy, macular degeneration (including age related macular degeneration and including both wet and dry forms), corneal diseases including corneal graft rejection, corneal injury, corneal scarring, corneal ulceration, corneal haze, keratoconus, glaucoma (preferably open angle glaucoma), myopia, ocular glaucoma, ocular hypertension, ocular vessel damage, angiogenesis, eye fibrosis (e.g. anterior subcapsular fibrosis, posterior subcapsular opacities, posterior capsular opacities, corneal haze after laser surgery, subconjunctival scarring after glaucoma surgery), proliferative vitreoretinopathy (PVR), bacterial ocular infections including hordeolum and ptilosis.

Ear diseases, encompassing, but not limited to, disorders such as Meniere’s disease, inflammation of the middle ear, inflammation of the external auditory canal and acute hearing loss.

Mouth, throat and respiratory diseases, comprising, without limitation, disorders such as inflammation of the oral mucosa and gums including aphta and stomatitis, parodontitis, epiglottitis, pharyngitis, laryngotracheitis, tonsillitis, common cold, angina, rhinitis including seasonal allergic rhinitis or perennial allergic rhinitis, rhinorrea, sinusitis of whatever type, etiology or pathogenesis or sinusitis that is a member selected from the group consisting of purulent or nonpurulent sinusitis, acute and chronic sinusitis and ethmoid, frontal, maxillary or sphenoid sinusitis, expectoration, pneumoconiosis of whatever type or genesis, including for example aluminosis, anthracosis, asbestosis, chalicosis, siderosis, silicosis, tabacosis and, in particular, byssinosis, bronchitis, cough, trachitis, congestion, pneumonia, eosinophilc lung infiltrate, chronic eosinophilic pneumonia, idiopathic pulmonary fibrosis and other fibrotic lung diseases, treatment related fibrotic lung disease e.g. related to radiation, methotrexate, chemotherapy, amiodarone or nitrofurantoin, sarcoidosis, acute respiratory distress syndrome (ARDS), severe acute respiratory syndrome (SARS), Coronavirus disease 2019 (COVID-19), bronchoconstriction, asthma of whatever type, etiology, or pathogenesis, or asthma that is a member selected from the group of atopic asthma, non-atopic asthma, allergic and non-allergic asthma, extrinsic asthma caused by environmental factors, intrinsic asthma caused by pathophysiologic disturbances, bronchial asthma, IgE-mediated asthma, essential asthma and essential asthma of unknown or inapparent cause, true asthma, emphysematous asthma, exercise-induced asthma, occupational asthma, infective asthma caused by bacterial, fungal, protozoal or viral infection, incipient asthma, wheezy infant syndrome, bronchial hyperreactivity, chronic obstructive pulmonary disease (COPD), COPD that is characterized by irreversible, progressive airways obstruction, acute respiratory distress syndrome (ARDS) and exacerbation of airways hyperreactivity consequent to other drug therapy, dyspnea, hyperoxic alveolar injury, pulmonary emphysema, pleurisy, tuberculosis, exposure to high altitude i.e. acute mountain sickness and preferably high altitude pulmonary edema (HAPE), resistant cough, bronchial hyporeactivity.

Gastrointestinal diseases, including, without limitation, disorders including esophagitis, gastritis, irritable stomach, gastric and duodenal ulcer, ileus, colon irritable, inflammatory bowel diseases including Crohn’s disease and ulcerative colitis, enteritis, hypertensive gastro- and colopathy, colitis, peritonitis, appendicitis, rectitis, gastrointestinal hemorrhage caused by a portal hypertension, collateral circulation or hyperemia, postgastrectomy dumping-syndrome, digestion discomfort, diarrhea, hemorrhoids, worm diseases, abdominal colic and colic of parts of the gastrointestinal system.

Liver, gallbladder and pancreatic diseases, encompassing, but not limited to, disorders such as hepatitis, cirrhosis of the liver, liver fibrosis (e.g. due to viral (HBV/HCV) infections, toxins (alcohol), fatty liver, bile stasis, hypoxia), portal hypertension, hepatorenal syndrome, hepatogenic edema, non-malignant ascites, cholangitis, cholecystitis, acute and chronic pancreatitis, and biliary colic.

Urinary tract and kidney diseases, including, without limitation, urinary tract infections such as acute and chronic cystitis, interstitial cystitis, irritable bladder, overactive bladder, incontinence including but not limited to stress-, urge and reflex incontinence, benign prostate hyperplasia, chronic renal disease, urethritis, inflammatory kidney diseases including glomerulonephritis, glomerular disease of the kidney, interstitial nephritis, pyelonephritis, diuresis, proteinuria, natriuresis, calciuresis, disorders of water balance, disorders of electrolyte balance, disorders of acid-base balance and renal colic, renal fibrosis, chronic renal allograft dysfunction, contrast-induced nephropathy.

Diseases of male genitale organs and female genitale organs, including, without limitation, altered sperm mobility, male infertility, orchitis, prostatitis, prostate enhancement, mastitis, inflammatory pelvis diseases, vaginal infections and pain, adnexitis, colpitis, soft ulcus, syphilis, clap and ovarian hyperstimulation syndrome.

Diseases of the hormone system, including, without limitation, menstrual disorders and pain, climacteric disturbance, emesis, premature uterine contractions, premature labor, endometriosis, endometritis, myoma, pre-eclampsia.

Metabolic diseases, including, without limitation, disorders such as diabetes, including non-insulin dependent diabetes mellitus, diabetic retinopathy, diabetic macular edema, diabetic nephropathy and diabetic neuropathy, insulin resistance and diabetic ulceration, diseases of the proteo- and purine metabolism such as gout and disorder of lipometabolism, hypoglycemia.

Cardiovascular diseases, including, without limitation, disorders including vascular permeability, vasodilation, hyperemia, peripheral circulatory disorders, cardiac volume overload, arterial circulatory disorders including aortic aneurysm, abdominal aortic aneurysm, brain aortic aneurysm, hypertension, intradialytic induced hypotension and hypotension associated with sepsis, restenosis after percutaneous transluminal coronary angioplasty, atherosclerosis including atherosclerotic plaque rupture, hemangioma, angiofibroma, venous disorders such as thrombosis, varicosity, phlebitis, thrombophlebitis, phlebothrombosis, cardiopathy, congestive heart failure, coronary heart disease, carcinoid syndrome, angina pectoris, cardiac dysrhythmias, inflammatory heart diseases including endocarditis, pericarditis and constrictive pericarditis, myocarditis, myocardial infarct, postmyocardial infarction syndrome, left ventricular dilation, post ischemic reperfusion injury, shock and collapse including septic, allergic, post traumatic and hemodynamic shock, amniotic fluid embolism, systemic inflammatory response syndrome (SIRS) including SIRS caused by heartlung bypass during surgery, sepsis and internal and external complications during cardiopulmonal bypass surgery (including but not limited to adverse hemodynamic effects following protamine sulfate reversal of heparine.

Blood diseases, including, without limitation, disorders such as coagulation, disseminated intravascular coagulopathy, hemorrhage, hemorrhagic diathesis, hypercholesterolemia and hyperlipemia, hypovolemic shock, paroxysmal nocturnal haemoglobinuria.

Lymphatic diseases, including, without limitation, splenomegaly, lymphangitis, lymphadenitis and hyperplastic adenoids.

Disorders of the central nervous system, including, without limitation, disorders such as inflammatory diseases of the central nervous system including encephalitis, meningitis, encephalomyelitis, meningoencephalitis, hydrocephalus, amyotrophic lateral sclerosis, spinal cord trauma, spinal cord edema, demyelinating diseases of the nervous system, multiple sclerosis, acute and chronic neuro-degenerative disorders including aging, Alzheimer’s disease and Parkinson’s disease, multiple sclerosis, myalgic Encephalomyelitis/Chronic Fatigue Syndrome, neuritis, and peripheral neuropathy, depressions, anorexia, anxiety and schizophrenia, sleep disorders.

Brain disorders, including, without limitation, disorders such as nootropic or cognition enhancement, cerebral amyloid angiopathy, stroke, head and brain trauma, traumatic brain injury, brain tumor, cerebral heat damage, cerebral ischemia, cerebral hemorrhage, post traumatic and post ischemic cerebral edema, general brain edema, acute mountain sickness and preferably high altitude cerebral edema (HACE), cytotoxic brain edema, vasogenic brain edema, post-surgical brain edema, brain edema associated with metabolic diseases, increase of permeability of blood-brain barrier or blood-brain tumor barrier.

Musculoskeletal system diseases, including, without limitation, disorders such as inflammatory musculoskeletal disorders, arthrosis, osteoarthrosis, osteoarthritis, chondroporosis after joint trauma or relatively long immobilization of a joint after meniscus or patella injuries or torn ligaments, rheumatoid arthritis of whatever type, etiology, or pathogenesis including acute arthritis, acute gouty arthritis, chronic inflammatory arthritis, degenerative arthritis, infectious arthritis, Lyme arthritis, proliferative arthritis, vertebral arthritis, septic arthritis, psoriatic arthritis, chronic polyarthritis, rheumatism, Sjogren’s syndrome, systemic lupus erythematosus, lumbago, spondylitis, spondylarthritis, ankylosing spondylitis, osteomyelitis, sprain, teno-synovitis, inflammation-induced bone resorption, fracture or the like, osteoporosis, musculoskeletal pain and hardening, spinal disk syndrome.

Allergy disorders, including, without limitation, disorders such as general allergic reactions, food allergy, anaphylactic shock, allergic contact hypersensitivity, allergic skin reactions, allergic asthma, vernal conjunctivitis and seasonal or perennial allergic rhinitis.

Pain, including, without limitation, centrally and peripherally mediated pain, vascular pain, visceral pain, inflammatory mediated pain, neuralgic pain, referred pain, nociceptive pain, reflectory pain, psychosomatic pain, acute pain such as caused by acute injury, trauma or surgery of bones, muscle, tissue, soft tissue, organs, opioid induced hyperalgesia, pain after insect bites, post-stroke pain syndrome, post-surgery pain, progressive disease related pain, chronic pain such as caused by neuropathic pain conditions (including but not limited to complex regional pain syndrome, causalgia, morbus sudeck, reflex sympathetic dystrophy, diabetic peripheral neuropathy, post-herpetic neuralgia, trigeminal neuralgia, cancer-related pain, pain associated with rheumatoid arthritis, osteoarthritis, teno-synovitis, gout, menstruation and angina, fibromyalgia, ocular pain, back pain, headache, cluster headache, tension headache, migraine, inflammatory pain, which may be associated with acute inflammation or chronic inflammation. Inflammatory pain includes but is not limited to neuropathic pain, ischemic pain, pain induced by arthritis, muscle pain induced by acute or chronic inflammation, neuralgia caused by acute or chronic inflammation, hyperalgesia. Also chemotherapy-induced peripheral neuropathy, hyperalgesia, opioid-induced hyperalgesia and fever. Furthermore, compounds of the invention are useful as analgesic agent for use during general and monitored anesthesia.

Infectious diseases, including, without limitation, diseases including those mediated by bacteria, viruses, fungi, parasites, protozoa, prions or mycobacterial infections. Particularly, the present invention is useful for the treatment of bacterial infections caused by Streptococcus, Escherichia, Salmonella, Staphylococcus, Klebsiella, Moracella, Haemophilus and Yersinia. Examples of bacterial infections intended to be within the scope of the present invention include, but are not limited to diseases such as pestis, sepsis, epidemic typhus, food poisoning, tetanus, scarlet red, whooping cough, diphtheria. Examples of viral infections intended to be within the scope of the present invention include, but are not limited to diseases such chickenpox and herpes zoster, AIDS, influenza, dengue virus fever, SARS-CoV-2 disease (COVID-19), hantavirus disease, small pox, and children diseases such as measles, rubella, mumps, acute anterior poliomyelitis. The present invention is useful for the treatment of protozoa and parasites infections caused by Schistosoma mansoni, Dermatofagoides farinae, Trypanosoma cruzi, Leishmania, and Plasmodium inducing Malaria. Examples of prion infections intended to be within the scope of the present invention include, but are not limited to diseases such bovine spongiform encephalopathy (BSE), Creutzfeldt Jacob disease and kuru, dengue fever, hemorrhagic fever.

Inflammatory disorders, including, without limitation, disorders such as acute-phase reaction, local and systemic inflammation and inflammation caused by other diseases whatever type, etiology or pathogenesis and caused by those inflammatory diseases specified within this application.

Injuries: Within the present application the term "injuries" encompasses, but is not limited to, multiple trauma, head and brain trauma, hypertensive tissue injury, lung injuries, external, internal and surgery wounds, burn injury including but not limited to thermal injury, electrical injury, chemical burns, cold injury, ionizing radiation, and sun burns.

Immunology disorders, including, without limitation, disorders such as hyperesthesia, autoimmune disorders, graft rejection in transplantation, transplant toxicity, granulomatous inflammation / tissue remodelling, myasthenia gravis, immunosuppression, immune-complex diseases, over- and underproduction of antibodies, vasculitis, delayed graft function, lupus.

Cancers, including, without limitation, disorders such as solid tumor cancer including breast cancer, lung cancer (non-small-cell lung cancer and small-cell lung cancer), prostate cancer, cancers of the oral cavity and pharynx (lip, tongue, mouth, pharynx), esophagus, stomach, small intestine, large intestine, colon, rectum, gallbladder and biliary passages, pancreas, larynx, lung, bone, osteosarcoma, connective tissue, skin cancer including Kaposi’s syndrome, melanoma and skin metastasis, epidermoid cancer, basal cell carcinoma, cervix uteri, corpus endometrium, cancer of ovary, testis, bladder, ureter and urethra, kidney, eye, brain and central nervous system, pseudotumor cerebri, sarcoma, sarcoid, thyroid and other endocrine glands (including but not limited to carcinoid tumors), Hodgkin’s disease, non-Hodgkin’s lymphomas, multiple myeloma, hematopoetic malignancies including leukemias and lymphomas including lymphocytic, granulocytic and monocytic lymphomas, tumor invasion, metastasis, ascites, tumor growth and angiogenesis.

Hereditary diseases, including, without limitation, disorders such as hereditary angioedema and angioneurotic edema, chondrocalcinosis, Huntington’s disease, mucoviscidosis.

Edema, as used herein, includes, but is not limited to, general edema, inlcuding any form and/or type of angioedema (AE), and edema caused by inflammation, Factor XII deficiency-induced edema, other drugs, e.g. drug induced angioedema, including but not limited to angiotensin-converting enzyme inhibitor-induced angioedema, drug-induced angioedema, thrombolytic therapy induced angioedema, infection, burns, injuries, trauma, frostbite, surgery, distorsions, fractures, exposure to high altitude (e.g. high altitude pulmonary edema (HAPE) and high altitude cerebral edema (HACE)), hereditary, autoimmune and other diseases and disorders, particularly but not limited to those disorders specified in this application, stress-induced edema (pronounced swelling) of gut

Capillary leak syndrome(s), includinge(s), without limitation, systemic capillary leak syndrome in sepsis, burn, allergy, drug/toxin-induced conditions, organ transplantation and IL-2 cytokine therapy.

In a preferred embodiment, the liquid pharmaceutical composition or capsule is used in the acute or chronic

treatment of angioedema (AE), including hereditary angioedema (HAE), acquired angioedema (AAE), bradykinin-mediated non-histaminergic idiopathic angioedema, allergic angioedema, or drug-induced angioedema, or bradykinin-mediated angioedema of unidentified cause. Hereditary angioedema (HAE) is a disorder that manifests in recurring attacks of severe swelling. The swelling often affects the arms, legs, face, intestinal tract, and also the airways. If the intestinal tract is affected, abdominal pain and vomiting may occur. Swelling of the airways may result in its bronchial obstruction and breathing difficulties. Acute attacks typically last for several days, and HAE patients experience attacks at a frequency of about every two weeks. The HAE can be of any type, including type I HAE, type II HAE, or type III HAE, preferably type I HAE or type II HAE.

According to a further preferred embodiment, the liquid pharmaceutical composition or capsule is used in the treatment of a prodrome or an acute attack of angioedema of a subject suffering from, for example, hereditary angioedema. As mentioned previously, the liquid pharmaceutical composition according to the invention shows a remarkable rate of release of the bradykinin B2 receptor antagonist of Formula 1, in spite of the compound's large molecular size and poor solubility in physiological liquids. With such rapid delivery of the active ingredient into the blood stream of the patient, the composition is particularly advantageous when used in the management of acute attacks or episodes of severe swelling that require a quick onset of drug action.

In a further embodiment, the liquid pharmaceutical composition or capsule is used in treatments comprising the oral administration of the respective composition or capsule once or twice daily, preferably over a period of at least two weeks. While the composition provides rapid drug release, almost like an injection, its oral administration is much more convenient than an injection, which make such regimen also particularly advantageous.

Further aspects of the invention, embodiments, optional features and preferences will become clear on the basis of the Examples and the patent claims.

Further definitions

For clarity, some further definitions of terms are given which are used throughout the description and claims. The definitions should be used to determine the meaning of the respective expressions unless the context requires a different meaning.

The terms ’a’ or ’an’ do not exclude a plurality, i.e., the singular forms ’a’, ’an’ and ’the’ should be understood as to include plural referents unless the context clearly indicates or requires otherwise. In other words, all references to singular characteristics or limitations of the present disclosure shall include the corresponding plural characteristic or limitation, and vice versa, unless explicitly specified otherwise or clearly implied to the contrary by the context in which the reference is made. The terms ’a’, ’an’ and ’the’ hence have the same meaning as ‘at least one’ or as ’one or more’ unless defined otherwise. For example, reference to ’an ingredient’ includes mixtures of ingredients, and the like.

The terms ’about’ or ’ca.’ will compensate for variability allowed for in the pharmaceutical industry and inherent in pharmaceutical products, such as differences in content due to manufacturing variation and/or time-induced product degradation. The term allows for any variation, which in the practice of pharmaceuticals would allow the product being evaluated to be considered bioequivalent in a subject to the recited strength of a claimed product.

The terms ’active agent’, ‘therapeutic agent’, ’active pharmaceutical ingredient (API)’, ’active principle’, ’drug’, ’bioactive agent’, are used synonymously and refer to a compound or combination of compounds which are pharmaceutically active against an undesired condition.

The term ’composition’ refers to any type of composition in which the specified ingredients may be incorporated, optionally along with any further constituents.

The term ’compound’ refers to a chemical substance, which is a material consisting of molecules having essentially the same chemical structure and properties. For a small molecular compound, the molecules are typically identical with respect to their atomic composition and structural configuration. For a macromolecular or polymeric compound, the molecules of a compound are highly similar but not all of them are necessarily identical.

The terms ’comprise’, ’comprises’ and ’comprising’ and similar expressions are to be construed in an open and inclusive sense, as ‘including, but not limited to’.

The terms ’essentially’, ’about’, ’approximately’, ’substantially’ and the like in connection with an attribute or value include the exact attribute or the precise value, as well as any attribute or value typically considered to fall within a normal range or variability accepted in the technical field concerned. For example, ’essentially free of water’ means that no water is deliberately included in a composition but does not exclude the presence of residual moisture.

The term ’essentially consisting of’ refers to compositions or dosage forms where no further components are added other than those listed. Nevertheless, very small amounts of other materials may potentially be present, such as material inherent impurities. Furthermore, when referring to e.g., ’essentially consisting of A, B, C and optionally D.’ this means that no further components are added to a composition or dosage form other than A, B, C and D, with D being an optional component (i.e., not mandatory) in said composition or dosage form.

The term ’essentially free’ refers to a composition that contains less than a functional amount of the respective

ingredient, typically less than 1 % by weight, preferably less than 0.1 % or even 0.01 %, and including zero percent by weight of the respective ingredient.

EXAMPLES

Example 1: Liquid compositions A-H

Several liquid compositions based on propylene glycol monocaprylate, polyoxyl castor oil, and propylene glycol (see **Tables 1A and 1B)** were prepared by weighing and mixing the respective liquid constituents and then combining the liquid mixture with the specified amount of the active ingredient such as to allow dissolution of the active ingredient (API) in the liquid mixture. As API, the compound (S)-N-(1-deutero-1-(3-chloro-5-fluoro-2-((2-methyl-4-(1-methyl-1H-1,2,4-triazol-5-yl)quinolin-8-yloxy)methyl)phenyl)ethyl)-2-(difluoromethoxy)acetamide monohydrate was used. The amount or content of the API is specified as monohydrate. Accordingly, the liquid compositions comprised the specified amount of the API monohydrate. As propylene glycol monocaprylate, a commercial grade of a propylene glycol monocaprylate type II (Capryol® 90) was used. As polyoxyl castor oil, a commercial grade of a polyoxyl 40 hydrogenated castor oil (Kolliphor® RH40) was used.

Table 1A

| Ingredient | A | B | C | D |
|---|---|---|---|---|
| API [mg/g] | 30 | 20 | 20 | 20 |
| Propylene glycol monocaprylate [parts by weight] | 20 | 20 | 30 | 40 |
| Polyoxyl castor oil [parts by weight] | 70 | 70 | 60 | 50 |
| Propylene glycol [parts by weight] | 10 | 10 | 10 | 10 |

Table 1B

| Ingredient | E | F | G | H |
|---|---|---|---|---|
| API [mg/g] | 50 | 20 | 50 | 50 |
| Propylene glycol monocaprylate [parts by weight] | 50 | 50 | 60 | 50 |
| Polyoxyl castor oil [parts by weight] | 40 | 40 | 30 | 30 |
| Propylene glycol [parts by weight] | 10 | 10 | 10 | 20 |

In result, it was observed that these compositions A-H incorporated the active ingredient in fully dissolved form, yielding a clear, opalescent or cloudy solution, without any solid residues that could indicate undissolved API.

Example 2: Dilution of liquid compositions A-H in aqueous medium

Compositions A-H were tested for their ability to maintain the API in its dissolved state upon dilution with an aqueous medium. For this purpose, a simple surrogate for gastric fluid, i.e. water acidified to pH 3 with hydrochloric acid solution, was added to samples of the compositions such as to dilute them by the factor of 10, i.e. to the 10-fold weight. The diluted samples were stored at room temperature for 3 hours.

Upon dilution, all compositions A-H formed emulsions or microemulsions that were physically stable, as could be observed visually, without any separation of the two liquid phases. Accordingly, the compositions were found to represent SEDDS or SMEDDS, as defined herein. Moreover, the diluted samples showed no indications of drug precipitation. In other words, the compositions were able to stabilise the active ingredient in fully dissolved form even when diluted with a significant excess of water.

Moreover, the diluted sample obtained from composition E was stored at room temperature for several further days and was remarkably stable: Even after 5 days, the sample was still in the form of an opalescent microemulsion with very little precipitation, showing that this composition is particularly advantageous and capable of solubilising the active ingredient such as to enable rapid absorption and a quick onset of action.

Example 3: Performance robustness of liquid composition

As it is known that many initially promising SMEDDS formulations of various drug substances eventually fail

during commercial product development because of inconsistent performance, or poor robustness of performance, a representative liquid composition according to the invention, i.e. composition E of Example 1, was subjected to a series of performance robustness tests

*Dilution in FaSSIF-V2*

Upon therapeutic administration of the liquid composition (e.g. by means of a soft capsule) it is possible that the composition is initially diluted with gastrointestinal fluid. To simulate this scenario, samples of the composition were mixed with FaSSIF-V2 (Fasted State Simulated Intestinal Fluid V2), a widely recognised biorelevant surrogate of gastrointestinal fluid, such as to achieve dilution ratios of 1/10 and 1/100, respectively. For comparison, a further series of samples were diluted with water, using the same dilution ratios. The diluted samples, which were all opaque microemulsions (also referred to as nanoemulsions in view of their sub-micron droplet sizes), were kept at 37°C. Emulsion droplet sizes were measured immediately upon dilution and after 6 hours, using a Malvern Zetasizer Nano ZS.

In result, it was found that all dilution samples represented sub-micron emulsions with z-average particle diameters in the range of about 50 to 200 nm. Minor differences were observed in that samples with a dilution ratio of 1/10 showed slightly larger droplet sizes than at a dilution ratio of 1/100, both initially and after 6 hours (e.g. 197 nm versus 119 nm for FaSSIF-V2). Moreover, storage over 6 hours led to some increase in droplet size for all dilutions. While this effect was slightly more pronounced in the case of FaSSIF-V2 compared to water as diluent, it was surprising to find that even after 6 hours at 37°C the dilutions in FaSSIF-V2 represented sub-micron emulsions, thus indicating a remarkable robustness of the formulation with respect to its performance, especially in view of the fact that FaSSIF-V2 is not only a buffer system but also comprises the surfactants sodium taurocholate and lecithin. The z-averages of the droplets measured in this series of experiments are summarised in Table 2 below.

Table 2

| Diluent (dilution ratio) | Droplet size (nm) at $t_0$ | Droplet size (nm) after 6 h |
|---|---|---|
| Water (1/10) | 93 | 102 |
| Water (1/100) | 50 | 64 |
| FaSSIF-V2 (1/10) | 80 | 197 |
| FaSSIF-V2 (1/100) | 58 | 119 |

*Dispersion in various media*

Three to five drops of composition E were separately added to 10 mL of each of the following media at 37°C: water, hydrochloric acid 0.01 n, phosphate buffer pH 6.8, simulated gastric fluid (SGF), Fasted State Simulated Intestinal Fluid (FaSSIF), and Fed State Simulated Intestinal Fluid (FeSSIF). The samples were visually inspected for indications of drug precipitation immediately upon mixing, after inverting, and after storage over 2 to 6 hours. In result, no signs of precipitation were observed.

*Temperature cycling*

Aliquots of about 5 g of liquid composition E were filled into vials and stored under refrigeration (2°C to 8°C) for approx. 24 hours. The vials were then visually inspected, in particular with respect to any drug precipitation or phase separation. Subsequently, the vials were stored at elevated temperature (30°C to 40°C) for another period of approx. 24 hours and inspected again. The cycling between the two temperature conditions was performed for six days. At no point in time, the visual inspection revealed any changes of the formulation, in particular no drug precipitation or phase separation.

Example 4: Liquid compositions I-N

A further series of liquid compositions based on propylene glycol monocaprylate, polyoxyl castor oil, propylene glycol and further liquid excipients (see Table 2) were prepared as described in Example 1. Again the compound (S)-N-(1-deutero-1-(3-chloro-5-fluoro-2-((2-methyl-4-(1-methyl-1H-1,2,4-triazol-5-yl)quinolin-8-yloxy)methyl)phenyl)ethyl)-2-(difluoromethoxy)acetamide monohydrate was used as API, and the same grades of propylene glycol monocaprylate and polyoxyl castor oil were used. For the excipient, caprylocaproyl polyoxyl-8 glycerides, also referred to as caprylocaproyl macrogol-8 glycerides, a commercially available grade (Labrasol®) was used.

Table 3

| Ingredient | I | K | L | M | N |
|---|---|---|---|---|---|
| API [mg/g] | 20 | 50 | 50 | 50 | 50 |
| Propylene glycol monocaprylate [parts by weight] | 20 | 50 | 50 | 60 | 50 |
| Polyoxyl castor oil [parts by weight] | 59.5 | 30 | 30 | 30 | 30 |
| Propylene glycol [parts by weight] | 10 | 10 | 20 | 10 | 20 |
| Caprylocaproyl polyoxyl-8 glycerides [parts by weight] | 10.5 | 0 | 0 | 0 | 0 |
| Ethanol [parts by weight] | 0 | 5 | 5 | 7.5 | 7.5 |

Upon visual inspection, it was found that also these compositions I-N were able to incorporate the active ingredient in fully dissolved form, yielding a clear, opalescent or cloudy solution, without any solid residues that could indicate undissolved API. This Example also indicates that, in addition to propylene glycol monocaprylate, polyoxyl castor oil, and propylene glycol, further excipients such as a further organic solvent (as represented by ethanol) and a further surfactant (as represented by the caprylocaproyl polyoxyl-8 glycerides) may be incorporated.

Example 5: Stability of liquid composition

Aliquots of liquid composition E were filled into glass vials and stored at different temperature and humidity conditions including 25°C/60% r.h. Samples were drawn and tested at different time intervals up to 36 months, including for drug content (assay) and chemical impurities, but also for physical appearance, water content and emulsification behaviour.

In result, no significant chemical or physical changes were seen. Even after 36 months, the composition was a clear, lightly brownish solution that showed emulsification, but no precipitation or phase separation after dilution with water, Remarkably, the drug content after 36 months was nearly identical to the initial value (94.1% versus 94.5% of label claim) and the total impurity content only showed a very minor increase (from 1.65% to 2.00%). These results not only promise a commercially attractive product shelf life, but are also very surprising in view of the fact that liquid SMEDDS formulations which comprise an active ingredient in fully dissolved, amorphous form within a mixture of highly functional excipients are typically much more prone to drug degradation than conventional pharmaceutical formulations.

Example 6: Dilution of liquid compositions I-N in aqueous medium

In analogy to Example 2, compositions I-N were tested for their ability to maintain the API in its dissolved state upon dilution with an aqueous medium. Again, all compositions spontaneously formed emulsions or microemulsions that were physically stable and showed no liquid phase separation. Also these compositions were thus found to represent SEDDS or SMEDDS. None of the diluted samples showed any indications of drug precipitation.

Comparative examples

Several other excipients and excipient combinations that are commonly used for preparing SEDDS or SMEDDS formulation of other active ingredients were tested with the bradykinin B2 receptor antagonist according to Formula 1 without success. For example, incorporating (S)-N-(1-deutero-1-(3-chloro-5-fluoro-2-((2-methyl-4-(1-methyl-1H-1,2,4-triazol-5-yl)quinolin-8-yloxy)methyl)phenyl)ethyl)-2-(difluoromethoxy)acetamide monohydrate in mixtures of:

(a) glyceryl caprylate/caprate (Capmul® MCM) and caprylocaproyl polyoxyl-8 glycerides (Labrasol®) at a weight ratio of 50:50;
(b) glyceryl caprylate/caprate (Capmul® MCM), caprylocaproyl polyoxyl-8 glycerides (Labrasol®) and ethanol at a weight ratio of 50:40:10;
(c) propylene glycol monocaprylate type II (Capryol® 90) and caprylocaproyl polyoxyl-8 glycerides (Labrasol®) at a weight ratio of 20:80; and
(d) propylene glycol monocaprylate type II (Capryol® 90), caprylocaproyl polyoxyl-8 glycerides (Labrasol®) and propylene glycol at various weight ratios,

led not only to quick precipitation of the active ingredient upon dilution with acidified water, but also to liquid-liquid phase separation, i.e. the physical destruction of the emulsion or microemulsion system and its conversion into two separate, non-dispersed liquid phases.

Moreover, dissolving the same compound of Formula 1 in other commonly used excipient mixtures:

(e) propylene glycol monocaprylate type II (Capryol® 90) and polyoxyl 40 hydrogenated castor oil (Kolliphor® RH40) at a weight ratio of 40:60;
(f) caprylocaproyl polyoxyl-8 glycerides (Labrasol®) and glyceryl caprylate/caprate (Capmul® MCM) at a weight ratio of 95:5; and
(g) polyoxyl 40 hydrogenated castor oil (Kolliphor® RH40); propylene glycol monocaprylate type II (Capryol® 90) and ethanol at a weight ratio of 70:20:10,

while not exhibiting liquid-liquid phase separation, showed rapid precipitation of the active ingredient.

Example 7: Preparation of soft gelatine capsules

Composition E of Example 1 was used as liquid fill material in the manufacture of soft gelatine capsules, using standard encapsulation equipment and techniques.
Two prototype capsule compositions (prototype E-1 and E-2) were prepared using the wet gelatine capsule shell composition shown in Table 4. A commercially available grade of sorbitol sirup, or liquid, partially dehydrated sorbitol, Polysorb® 85/70/00 (Roquette), was used.

Table 4

| Ingredient | E-1 (wt%) | E-2 (wt%) |
|---|---|---|
| Gelatine 195 Bloom (acid bone) | 44.83 | |
| Gelatine 150 Bloom (lime bone) | | 39.14 |
| Sorbitol sirup | 12.81 | |
| Propylene glycol | | 14.68 |
| Glycerol 99.5% | 12.81 | 12.47 |
| Purified water | 28.08 | 31.55 |
| Titanium dioxide | 1.48 | 1.13 |
| Red iron oxide | | 0.49 |
| Yellow iron oxide | | 0.54 |
| Lecithin (0.3 wt%) solution in medium chain triglycerides | q.s. | q.s. |

Upon visual inspection by an experienced technician, the soft capsule appearance was found to be very good for both capsule formulations. There were smooth surfaces and well-formed sealing areas. No stretching of gelatine and no particular defects were observed, so that the capsule shell compositions were found suitable for encapsulating liquid composition E.
The hardness of the capsules was tested and monitored over storage at various temperature and humidity conditions (25°C/60% rh; 30°C/65% rh; 40°C/75% rh) over a period of three months. It was found that only minor changes occurred that did not impact the overall properties of the capsules.

Example 8: Single dose pharmacokinetic study in monkeys

The pharmacokinetic properties of a liquid composition according to the invention after a single oral administration in Cynomolgus monkeys was studied and compared with those of a suspension of the same API in an aqueous carrier comprising methylcellulose (1 wt.%). The Test Formulation was based on composition E of Example 1, except that the concentration of the API was 5 mg/mL in essentially the same liquid vehicle as composition E. The Comparative Formulation comprised the same API at a concentration of 2 mg/mL, formulated as an aqueous drug suspension further comprising methylcellulose (1 wt.%).

Materials and methods:

The study was performed in 3 animals, in 2 phases separated by at least 5 days of wash-out, as follows:

Phase 1:

| | Dose level (mg/kg) | Dose volume (mL/kg) | API conc. (mg/mL) |
|---|---|---|---|
| Comparative Formulation | 10 | 5 | 2 |

Once Phase 1 was completed, monkeys were reallocated in Phase 2 in the same order.

Phase 2:

| | Dose level (mg/kg) | Dose volume (mL/kg) | API conc. (mg/mL) |
|---|---|---|---|
| Test Formulation | 10 | 2 | 5 |

Nominal dose levels were used for the pharmacokinetic evaluation.

The non-human primate was a particularly appropriate species for this study because in vitro pharmacology testing revealed that the API has a similar high antagonist potency for the human and the monkey B2 receptors, but has low antagonist potency for the dog, rat and mouse B2 receptors.

Blood samples for the pharmacokinetic evaluation were taken from all animals in both phases before dosing, as well as at 0.5, 1, 2, 3, 4, 6, 8 and 24 hours after dosing. All blood samples were collected within an interval strictly lower than 20 % of the nominal sampling time, and the theoretical sampling times were considered for the pharmacokinetic evaluation.

The pharmacokinetic parameters were determined from the individual plasma concentrations by non-compartmental analysis, using Kinetica™ 4.4.1 (Thermo Fisher). Plasma concentrations below the LLOQ (i.e. <1.2 ng/mL for test item) were taken as 0. Individual concentration versus time graphs in linear and semi-logarithmic scales were performed using Kinetica™ 4.4.1 **(Figures 1A-1D).**

Pharmacokinetic parameters, ratios, SD and CV (or delta %) were reported to 3 significant figures for numbers less than 100 and to the nearest integer for all numbers greater or equal to 100, with the exception of time values and n.

*Principal pharmacokinetic parameters*

Maximum plasma concentration (Cmax), sampling time of Cmax (Tmax), and sampling time of the last plasma concentration >LLOQ (Tlast) parameters were determined from the observations. The area under the plasma concentration-time curve from the time of dosing to the last quantifiable concentration was calculated using the linear up/logarithmic down trapezoidal rule (AUClast). At least 3 consecutive quantifiable concentrations had to be available for AUC calculation.

*Secondary pharmacokinetic parameters*

The linear regression coefficient (R) of the log-linear terminal phase of the concentration-time profile was calculated by Kinetica™ 4.4.1, where at least 3 data points other than Cmax were available. The coefficient of regression (R) was presented in absolute value and the period was determined as the span of time-points used to calculate the linear regression of the log-linear terminal phase. When the absolute value of R was greater than or equal to 0.8 and the period was twice greater than the half-life, the elimination rate constant value (k) of the linear regression could be used for further calculations and thus t1/2 was calculated after single administration, using the equation ln2/k.

The percentage of extrapolated AUC from Tlast to infinity was calculated by KineticaTM 4.4.1, using the following equation:

$$\text{AUCextra (\%)} = (\text{Clast} / \text{k}) \times 100 / \text{AUCinf}$$

When this extrapolation was below 20 % and the 2 above mentioned conditions (R and period) were fulfilled, the following parameters are presented after a single administration:

AUCinf: estimated area under the curve from time of dosing to infinity, calculated using the following equation:

$$AUCinf = AUClast + AUCextra$$

*Dose effect and comparison of analytes or formulations*

Dose proportionality: this effect was assessed graphically and by calculating the individual dose normalized Cmax and AUClast.

Comparison of formulation: this effect was assessed by calculating the individual Cmax and AUClast ratios between the Comparative Formulation and the Test Formulation.

*Results*

The pharmacokinetic parameters are listed in Table 5. Moreover, **Figure 1** shows the individual API plasma concentrations versus time in linear and semi-logarithmic scales. Figures 1A and 1B show the API plasma concentrations after administration of the API in the aqueous carrier comprising methylcellulose (1 wt.%). Figures 1C and 1D show the API plasma concentrations after administration of the API in the composition according to the invention.

Table 5: Mean pharmacokinetic parameters

| Analyte | Dose (mg/kg) | Formulation | Cmax (ng/mL) | AUClast (ng.h/mL) | Tmax (h) |
|---|---|---|---|---|---|
| API | 10 | Comparative Formulation (API suspension in 1 % methylcellulose) | 303 | 1237 | 0.5-1 |
| | | Test Formulation (API in Kolliphor RH40/ Capryol 90/PG 40:50:10 (w/w/w) formulation) | 551 | 3296 | 4 |

Exposure to the API was demonstrated in all animals.

The calculated parameters were very variable between animals: 67 % of the values had a CV or delta % above 30 %.

API plasma concentrations were quantifiable up to 24 hours after administration, except for one animal treated with the Test Formulation, for which no compound was detected at 24 hours **(Tables 6 and 7).** Maximum API plasma concentrations were observed between 0.5 and 3 hours after dosing with Comparative Formulation and between 1 and 4 hours after dosing with the Test Formulation **(Tables 6 and 7).**

Table 6: Mean pharmacokinetic parameters after administration to monkeys

| Dose (mg/kg) | Formulation | | Cmax (ng/mL) | Tmax (h) | AUClast (ng.h/mL) | AUCinf (ng.h/mL) | t1/2 (h) |
|---|---|---|---|---|---|---|---|
| 10 | Comparative Formulation (API suspension in 1% methylcellulose) | Mean | 303 | 1 [0.5 - 1] | 1237 | NA | NA |
| | | SD | 64.6 | NA | 440 | NA | NA |
| | | CV | 21.3 | NA | 35.5 | NA | NA |
| | | %n | 3 | 3 | 3 | 0 | 0 |
| | Test Formulation Formulation (API cremophor RH40/ Capryol 90/PG 40: 50:10 (w/w) formulation) | Mean | 551 | 4 [4 - 4] | 3296 | 4330 | 2.76 |
| | | SD | 299 | NA | 1918 | NA | NA |
| | | CV | 54.3 | NA | 58.2 | 35.1 | 2.84 |
| | | %n | 3 | 3 | 3 | 2 | 2 |

For the Comparative Formulation, no half-life values could be reported, therefore no conclusion could be drawn on elimination. For the Test Formulation, the half-life values were relatively similar irrespective of dose: individual values were between 2.72 and 4.57 hours **(Table 7).**

Table 7: Individual pharmacokinetic parameters after administration to monkeys

| Dose (mg/kg) | Formulation | Animal Identification # | Cmax (ng/mL) | Tmax (h) | Tlast (h) | AUClast (ng.h/mL) | AUCextra (%) |
|---|---|---|---|---|---|---|---|
| 10 | Comparative Formulation (API suspension in 1% methylcellulose) | M2151 | 346 | 0.5 | 24 | 1516 | 41.2 |
| | | M2152 | 334 | 1 | 24 | 1466 | NA |
| | | M2153 | 228 | 1 | 24 | 731 | 11.7 |
| | | **Mean** | **303** | **1** | **NA** | **1237** | **NA** |
| | | **SD** | **64.6** | **NA** | **NA** | **440** | **NA** |
| | Test Formulation (API cremophor RH40/ Capryol 90/PG 40:50:10 (w/w) formulation) | M2157 | 675 | 4 | 24 | 5069 | 0.420 |
| | | M2158 | 768 | 4 | 24 | 3557 | 0.361 |
| | | M2159 | 210 | 4 | 12 | 1261 | 29.2 |
| | | **Mean** | **551** | **4** | **NA** | **3296** | **NA** |
| | | **SD** | **299** | **NA** | **NA** | **1918** | **NA** |
| | | M2154 | 529 | 1 | 24 | 2033 | 25.0 |
| Formulation | Animal Identification # | \|R\| | Period (h) | AUCinf (ng.h/mL) | t1/2 (h) | Cmax/ dose | AUClast/ dose |
| Comparative Formulation (API suspension in 1% methylcellulose) | M2151 | 1.00 | 16.0 | NA | NA | 34.6 | 152 |
| | M2152 | NA | NA | NA | NA | 33.4 | 147 |
| | M2153 | 0.993 | 16.0 | NA | NA | 22.8 | 73.1 |
| | **Mean** | **NA** | **NA** | **NA** | **NA** | **30.3** | **124** |
| | **SD** | **NA** | **NA** | **NA** | **NA** | **6.46** | **44.0** |

(continued)

| Formulation | Animal Identification # | \|R\| | Period (h) | AUCinf (ng.h/mL) | t1/2 (h) | Cmax/ dose | AUClast/ dose |
|---|---|---|---|---|---|---|---|
| Test Formulation (API cremophor RH40/ Capryol 90/PG 40:50:10 (w/w) formulation) | M2157 | 0.996 | 18.0 | 5090 | 2.72 | 67.5 | 507 |
| | M2158 | 0.996 | 16.0 | 3570 | 2.80 | 76.8 | 356 |
| | M2159 | 0.962 | 6.00 | NA | NA | 21.0 | 126 |
| | **Mean** | **NA** | **NA** | **4330** | **2.76** | **55.1** | **330** |
| | **SD** | **NA** | **NA** | **NA** | **NA** | **29.9** | **192** |
| | M2154 | 0.519 | 22.0 | NA | NA | 5.29 | 20.3 |

*Comparison of formulations:* The systemic exposure of the API was much higher with Test Formulation than with Comparative Formulation. Mean Cmax and AUClast ratios (Comparative Formulation to Test Formulation) were 0.679 and 0.430, respectively **(Table 8).** In other words, the extent of oral bioavailability achieved by the Test Formulation was more than twice as high as that of the Comparative Formulation.

Table 8: Comparative Formulation / Test Formulation Cmax and AUClast ratios

| Analyte | Dose (mg/kg) | Animal Identification | Cmax ratio Comparative Formulation/ Test Formulation | AUClast ratio Comparative Formulation/ Test Formulation |
|---|---|---|---|---|
| API | 10 | M2151 / M2157 | 0.512 | 0.299 |
| | | M2152 / M2158 | 0.435 | 0.412 |
| | | M2153 / M2159 | 1.09 | 0.579 |
| | | *Mean* | *0.679* | *0.430* |
| | | *SD* | *0.357* | *0.141* |

*Mortality.* No mortality occurred during this study.

Example 9: Oral bioavailability in humans

The oral bioavailability, pharmacokinetic properties, and safety of a liquid composition according to the invention after a single oral administration was evaluated. The composition was identical to composition E of Example 1.

Methods: The composition was administered as an oral solution in a double-blind placebo-controlled single ascending dose first-in-human study in healthy volunteers. **Table 9** shows the experimental design of the study.

Table 9: Experimental design

| Dose | Part 1 (PK) | Part 2(PD) |
|---|---|---|
| 1 mg * | N=6 | |
| 2 mg * | N=6 | |
| 4.5 mg * | N=6 | |
| 12 mg * | N=6 | N=8 |
| 22 mg * | N=6 | N=8 |
| 22 mg§ | N=6 | |
| Placebo | N=12 | N=4 |
| * fasted condition | | |

(continued)

| Dose | Part 1 (PK) | Part 2(PD) |
|---|---|---|
| § after high caloric/ high fat (HCHF) breakfast | | |

Safety was assessed by physical examination, vital signs, adverse events, safety laboratory and electrocardiogram (ECG) until 72 h post-dosing. Plasma pharmacokinetic (PK) parameters were assessed until 72 h post-dosing.

Pharmacokinetics Results:

The composition was very rapidly absorbed and reached peak plasma levels within 30 to 60 minutes after dosing in all subjects under fasted conditions. The systemic exposure was dose proportional with a mean t1/2 ranging from 3.5 to 5.6 h between doses. Plasma levels for the API reached therapeutic efficacious threshold concentration (estimated EC50 2.4 ng/mL and EC85 13.8 ng/mL) within 15 min for all doses and were maintained for approximately 12 h with doses of 12 and 22 mg **(Figure 2).**

Administration of the 22 mg dose with a HCHF breakfast led to a 32% lower Cmax, a 49% higher AUClast, and a delay of median tmax by approximately 2 h. Plasma levels still reached levels expected to be therapeutically effective within 15 min and were maintained for more than 12 h **(Figure 3).** A summary of the observed pharmacokinetic parameters is provided in **Table 10.**

Table 10: Pharmacokinetic parameters in human subjects. For $C_{max}$, $C_{0.25h}$, $C_{12h}$, $AUC_{last}$, $T_{1/2}$, $V_Z/F$, and CL/F, the values shown are the means, standard deviations are shown in brackets. For $T_{max}$, the values shown are the medians, ranges are shown in brackets. * indicates fasted conditions. § indicates after HCHF.

| **Dose** | **1 mg *** | **2 mg *** | **4.5 mg *** | **12 mg *** | **22 mg *** | **22 mg §** |
|---|---|---|---|---|---|---|
| | N=6 | N=6 | N=6 | N=6 | N=6 | N=6 |
| $C_{max}$, ng/mL | 11.1 (4.03) | 19.8 (3.70) | 32.9 (7.66) | 97.3 (28.1) | 213 (49.5) | 145 (56.2) |
| $T_{max}$, h | 0.50 (0.25-1.00) | 0.75 (0.25-1.02) | 1.00 (0.50-1.00) | 0.50 (0.25-1.00) | 0,75 (0.25-1.02) | 3.00 (2.00-3.00) |
| $C_{0.25h}$, ng/mL | 5.99 (4.28) | 12.9 (8.10) | 13.0 (7.00) | 60.3 (40.6) | 143 (85.9) | 48.3 (53.9) |
| $C_{12h}$, ng/mL | 0.528 (0.670) | 0.810 (0.619) | 1.93 (1.87) | 5.58 (5.66) | 8.34 (4.24) | 19.6 (10.7) |
| $AUC_{last}$, ng.h/mL | 33.0 (25.9) | 66.0 (27.0) | 129 (56.5) | 369 (194) | 681 (113) | 1015 (490) |
| $T_{1/2}$, h | 3.49 (1.32) | 4.26 (1.91) | 4.36 (1.29) | 4.25 (0.831) | 5.61 (0.707) | 5.31 (1.54) |
| $V_Z/F$, L | 190 (95.7) | 181 (34.3) | 222 (43.7) | 235 (96.3) | 252 (30.9) | 180 (51.8) |
| CL/F, L/h | 42.3 (25.4) | 33.8 (11.9) | 37.6 (11.1) | 40.9 (20.7) | 31.5 (5.60) | 25.3 (9.77) |

These results not only confirm the remarkable characteristics of the composition as already indicated by the results of Example 8, showing that therapeutically relevant plasma levels are reliably achieved, but they also show a remarkably rapid absorption of the active ingredient into the systemic blood circulation, in spite of its very low water solubility. With such pharmacokinetic performance, the composition is clearly useful for the oral treatment of patients suffering from a disease or condition responsive to bradykinin B2 receptor modulation in general, and even for the treatment of acute outbreaks or symptoms that require an immediately effective intervention.

## Claims

1. A liquid pharmaceutical composition for oral administration comprising a bradykinin (BK) B2 receptor antagonist having a chemical structure according to Formula 1, or a stereoisomer, salt or solvate thereof:

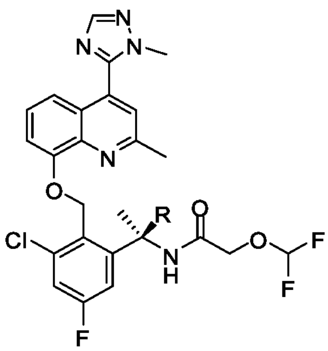

(Formula 1)

wherein R is deuterium or hydrogen;
wherein the BK B2 receptor antagonist is dissolved in a liquid vehicle comprising propylene glycol monocaprylate, polyoxyl castor oil, and propylene glycol.

**2.** The liquid pharmaceutical composition of claim 1, wherein the BK B2 receptor antagonist is a compound according to Formula 1, or a salt or solvate thereof, wherein R is deuterium.

**3.** The liquid pharmaceutical composition of claim 1 or 2, wherein the amount of propylene glycol monocaprylate in the liquid vehicle is 40-60 wt.%, based on the weight of the liquid vehicle.

**4.** The liquid pharmaceutical composition of any one of the preceding claims, wherein the amount of polyoxyl castor oil in the liquid vehicle is 30-50 wt.%, based on the weight of the liquid vehicle.

**5.** The liquid pharmaceutical composition of any one of the preceding claims, wherein the amount of propylene glycol is 2.5-15 wt.%, based on the weight of the liquid vehicle.

**6.** The liquid pharmaceutical composition of any one of the preceding claims, wherein the propylene glycol monocaprylate is a propylene glycol monocaprylate type II (USP/NF).

**7.** The liquid pharmaceutical composition of any one of the preceding claims, wherein the polyoxyl castor oil is a polyoxyl 40 hydrogenated castor oil (USP/NF).

**8.** The liquid pharmaceutical composition of any one of the preceding claims, wherein the content of propylene glycol monocaprylate is 50 wt.%, the content of polyoxyl castor oil is 40 wt.%, and the content of propylene glycol is 10 wt.%, based on the combined weight of propylene glycol mono caprylate, polyoxyl castor oil, and propylene glycol in the liquid vehicle.

**9.** The liquid pharmaceutical composition of any one of the preceding claims, exhibiting a content of the BK B2 receptor antagonist in the range from 5 mg to 100 mg per g, such as from 5 mg to 65 mg per g, or such as from 20 mg to 70 mg per g, of the liquid pharmaceutical composition.

**10.** The liquid pharmaceutical composition of any one of the preceding claims, essentially consisting of:

(a) based on the total weight of the liquid pharmaceutical composition, 0.5 to 6.5 wt.%, such as 5 wt.%, of a compound according to Formula 1, or a stereoisomer, salt or solvate thereof, wherein R is deuterium;
(b) based on the total weight of the liquid pharmaceutical composition, 93.5 to 99.5 wt.% of a liquid vehicle, said liquid vehicle essentially consisting of:

(i) 50 parts (in weight) of propylene glycol monocaprylate, such as propylene glycol monocaprylate type II (USP/NF);
(ii) 40 parts (in weight) of polyoxyl castor oil, such as 40 hydrogenated castor oil (USP/NF);
(iii) 5 to 10 parts (in weight) of propylene glycol; and optionally (iv) up to 5 parts (in weight) of water;

and optionally
(c) the balance being one or more further excipients dissolved or dispersed in the liquid vehicle.

**11.** A capsule for oral administration, comprising the liquid pharmaceutical composition of any one of the preceding claims.

**12.** The capsule of claim 11, wherein the capsule is a soft capsule.

**13.** The soft capsule of claim 12, comprising a capsule wall which comprises gelatine, water, and at least one plasticiser selected from propylene glycol, glycerol, sorbitol, sorbitan, sorbitol-based plasticiser mixtures, or any combinations thereof.

**14.** The liquid pharmaceutical composition of any one of the claims 1 to 10, or the capsule of any one of the claims 11 to 13, for use in the acute or chronic treatment of a subject suffering from a disease or condition responsive to BK B2 receptor modulation.

**15.** The liquid pharmaceutical composition for use according to claim 14, or the capsule for use according to claim 14, wherein the disease or condition responsive to BK B2 receptor modulation is edema, such as hereditary angioedema.

## Patentansprüche

**1.** Flüssige pharmazeutische Zusammensetzung zur oralen Verabreichung, umfassend einen Bradykinin (BK) B2-Rezeptor-Antagonisten mit einer chemischen Struktur gemäß Formel 1 oder ein Stereoisomer, Salz oder Solvat desselben:

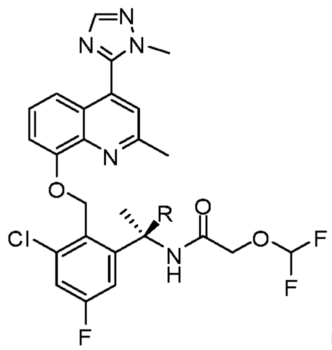

(Formel 1)

wobei R Deuterium oder Wasserstoff ist;
wobei der BK B2-Rezeptor-Antagonist in einem flüssigen Träger gelöst ist, der Propylenglykolmonocaprylat, Polyoxyl-Rizinusöl und Propylenglykol enthält

**2.** Die flüssige pharmazeutische Zusammensetzung nach Anspruch 1, wobei der BK-B2-Rezeptor-Antagonist eine Verbindung gemäß Formel 1 oder ein Salz oder Solvat desselben ist, wobei R Deuterium ist

**3.** Die flüssige pharmazeutische Zusammensetzung nach Anspruch 1 oder 2, wobei die Menge an Propylenglykolmonocaprylat in dem flüssigen Träger 40-60 Gew.-%, bezogen auf das Gewicht des flüssigen Trägers, beträgt.

**4.** Die flüssige pharmazeutische Zusammensetzung nach einem der vorhergehenden Ansprüche, wobei die Menge an Polyoxyl-Rizinusöl in dem flüssigen Träger 30-50 Gew.-%, bezogen auf das Gewicht des flüssigen Trägers, beträgt

**5.** Die flüssige pharmazeutische Zusammensetzung nach einem der vorhergehenden Ansprüche, wobei die Menge an Propylenglykol 2,5-15 Gew.-%, bezogen auf das Gewicht des flüssigen Trägers, beträgt.

**6.** Die flüssige pharmazeutische Zusammensetzung nach einem der vorhergehenden Ansprüche, wobei das Propylenglykolmonocaprylat ein Propylenglykolmonocaprylat vom Typ II (USP/NF) ist.

**7.** Die flüssige pharmazeutische Zusammensetzung nach einem der vorhergehenden Ansprüche, wobei das Polyoxyl-Rizinusöl ein Polyoxyl-40-hydriertes Rizinusöl (USP/NF) ist.

**8.** Die flüssige pharmazeutische Zusammensetzung nach einem der vorhergehenden Ansprüche, wobei der Gehalt an Propylenglykolmonocaprylat 50 Gew.-%, der Gehalt an Polyoxyl-Rizinusöl 40 Gew.-% und der Gehalt an Propylenglykol 10 Gew.-% beträgt, bezogen auf das kombinierte Gewicht von Propylenglykolmonocaprylat, Polyoxyl-Rizinusöl und Propylenglykol in dem flüssigen Träger.

**9.** Die flüssige pharmazeutische Zusammensetzung nach einem der vorhergehenden Ansprüche, die einen Gehalt des BK B2-Rezeptor-Antagonisten im Bereich von 5 mg bis 100 mg pro g, wie beispielsweise von 5 mg bis 65 mg pro g oder beispielsweise von 20 mg bis 70 mg pro g, der flüssigen pharmazeutischen Zusammensetzung aufweist.

**10.** Die flüssige pharmazeutische Zusammensetzung nach einem der vorhergehenden Ansprüche, welche im wesentlichen besteht aus:

(a) bezogen auf das Gesamtgewicht der flüssigen pharmazeutischen Zusammensetzung, 0,5 bis 6,5 Gew.-%, wie beispielsweise 5 Gew.-%, einer Verbindung gemäß Formel 1 oder eines Stereoisomers, Salzes oder Solvats derselben, wobei R Deuterium ist;
(b) bezogen auf das Gesamtgewicht der flüssigen pharmazeutischen Zusammensetzung, 93,5 bis 99,5 Gew.-% eines flüssigen Trägers, wobei der flüssige Träger im Wesentlichen besteht aus:

(i) 50 Teilen (in Gewicht) Propylenglykolmonocaprylat, wie beispielsweise Propylenglykolmonocaprylat vom Typ II (USP/NF);
(ii) 40 Teilen (in Gewicht) Polyoxyl-Rizinusöl, wie beispielsweise Polyoxyl-40-hydriertes Rizinusöl (USP/NF);
(iii) 5 bis 10 Teile (in Gewicht) Propylenglykol; und gegebenenfalls (iv) bis zu 5 Teile (in Gewicht) Wasser;

und wobei wahlweise
(c) der Rest ein oder mehrere weitere Hilfsstoffe ist/sind, der/die in dem flüssigen Träger gelöst oder dispergiert ist/sind.

**11.** Kapsel zur oralen Verabreichung, umfassend die flüssige pharmazeutische Zusammensetzung nach einem der vorhergehenden Ansprüche.

**12.** Die Kapsel nach Anspruch 11, wobei die Kapsel eine Weichkapsel ist

**13.** Die Weichkapsel nach Anspruch 12, umfassend eine Kapselwand, die Gelatine, Wasser und mindestens einen Weichmacher, der aus Propylenglykol, Glycerin, Sorbit, Sorbitan, Weichmachergemischen auf Sorbitbasis oder beliebigen Kombinationen derselben ausgewählt ist, umfasst

**14.** Die flüssige pharmazeutische Zusammensetzung nach einem der Ansprüche 1 bis 10 oder die Kapsel nach einem der Ansprüche 11 bis 13 zur Verwendung bei der akuten oder chronischen Behandlung eines Subjekts, das an einer Krankheit oder einem Zustand leidet, der auf eine BK B2-Rezeptormodulation anspricht.

**15.** Die flüssige pharmazeutische Zusammensetzung zur Verwendung gemäß Anspruch 14 oder die Kapsel zur Verwendung gemäß Anspruch 14, wobei die Krankheit oder der Zustand, die/der auf die BK B2-Rezeptormodulation anspricht, ein Ödem ist, wie beispielsweise ein hereditäres Angioödem.

## Revendications

**1.** Composition pharmaceutique liquide pour administration orale comprenant un antagoniste du récepteur B2 de la bradykinine (BK) ayant une structure chimique selon la formule 1, ou un stéréo-isomère, un sel ou un solvate de celle-ci:

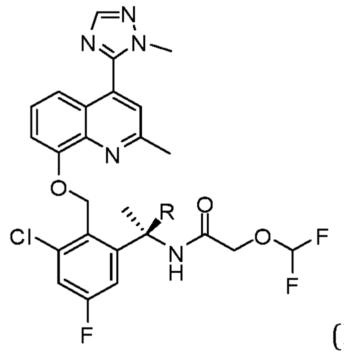

(Formule 1)

dans laquelle R représente le deutérium ou l'hydrogène;
dans lequel l'antagoniste du récepteur BK B2 est dissous dans un véhicule liquide comprenant du propylène glycol monocaprylate, de l'huile de ricin polyoxylée et du propylène glycol.

**2.** La composition pharmaceutique liquide de la revendication 1, dans laquelle l'antagoniste du récepteur BK B2 est un composé selon la formule 1, ou un sel ou un solvant de celui-ci, dans lequel R est le deutérium.

**3.** La composition pharmaceutique liquide de la revendication 1 ou 2, dans laquelle la quantité de monocaprylate de propylène glycol dans le véhicule liquide est de 40 à 60 % en poids, sur la base du poids du véhicule liquide.

**4.** La composition pharmaceutique liquide de l'une des revendications précédentes, dans laquelle la quantité d'huile de ricin polyoxylée dans l'excipient liquide est de 30 à 50 % en poids, sur la base du poids du véhicule liquide.

**5.** La composition pharmaceutique liquide de l'une des revendications précédentes, dans laquelle la quantité de propylène glycol est de 2,5 à 15 % en poids, sur la base du poids du véhicule liquide.

**6.** La composition pharmaceutique liquide de l'une des revendications précédentes, dans laquelle le monocaprylate de propylène glycol est un monocaprylate de propylène glycol de type II (USP/NF).

**7.** La composition pharmaceutique liquide de l'une quelconque des revendications précédentes, dans laquelle l'huile de ricin polyoxyle est une huile de ricin hydrogénée polyoxyle 40 (USP/NF).

**8.** La composition pharmaceutique liquide de l'une des revendications précédentes, dans laquelle la teneur en monocaprylate de propylène glycol est de 50 % en poids, la teneur en huile de ricin polyoxyle est de 40 % en poids et la teneur en propylène glycol est de 10 % en poids, sur la base du poids combiné du monocaprylate de propylène glycol, de l'huile de ricin polyoxyle et du propylène glycol dans véhicule liquide.

**9.** La composition pharmaceutique liquide de l'une quelconque des revendications précédentes, présentant une teneur en antagoniste du récepteur BK B2 comprise entre 5 mg et 100 mg par g, par exemple entre 5 mg et 65 mg par g, ou par exemple entre 20 mg et 70 mg par g, de la composition pharmaceutique liquide.

**10.** La composition pharmaceutique liquide de l'une quelconque des revendications précédentes, consistant essentiellement en:

(a) sur la base du poids total de la composition pharmaceutique liquide, 0,5 à 6,5 % en poids, par exemple 5 % en poids, d'un composé selon la formule 1, ou d'un stéréo-isomère, d'un sel ou d'un solvate de celui-ci, dans lequel R est le deutérium;
(b) sur la base du poids total de la composition pharmaceutique liquide, 93,5 à 99,5 % en poids d'un véhicule liquide, ledit véhicule liquide étant essentiellement constitué de:

(i) 50 parties (en poids) de monocaprylate de propylène glycol, par exemple le monocaprylate de propylène glycol de type II (USP/NF);
(ii) 40 parties (en poids) d'huile de ricin polyoxylée, par exemple l'huile de ricin hydrogénée 40 (USP/NF);
(iii) 5 à 10 parties (en poids) de propylène glycol; et éventuellement iv) jusqu'à 5 parties (en poids) d'eau;

et éventuellement
(c) le reste étant un ou plusieurs autres excipients dissous ou dispersés dans le véhicule liquide.

**11.** Capsule pour administration orale, comprenant la composition pharmaceutique liquide de l'une des revendications précédentes.

**12.** Capsule de la revendication 11, dans laquelle la capsule est une capsule molle.

**13.** La capsule molle de la revendication 12, comprenant une paroi de capsule qui comprend de la gélatine, de l'eau, et au moins un plastifiant choisi parmi le propylène glycol, le glycérol, le sorbitol, le sorbitan, les mélanges de plastifiants à base de sorbitol, ou toute combinaison de ceux-ci.

**14.** La composition pharmaceutique liquide de l'une des revendications 1 à 10, ou la capsule de l'une des revendications 11 à 13, pour une utilisation dans le traitement aigu ou chronique d'un sujet souffrant d'une maladie ou d'une condition répondant à la modulation du récepteur BK B2.

**15.** La composition pharmaceutique liquide pour utilisation selon la revendication 14, ou capsule pour utilisation selon la revendication 14, dans laquelle la maladie ou l'état répondant à la modulation du récepteur BK B2 est l'œdème, par exemple l'œdème angioneurotique héréditaire.

FIG. 1A

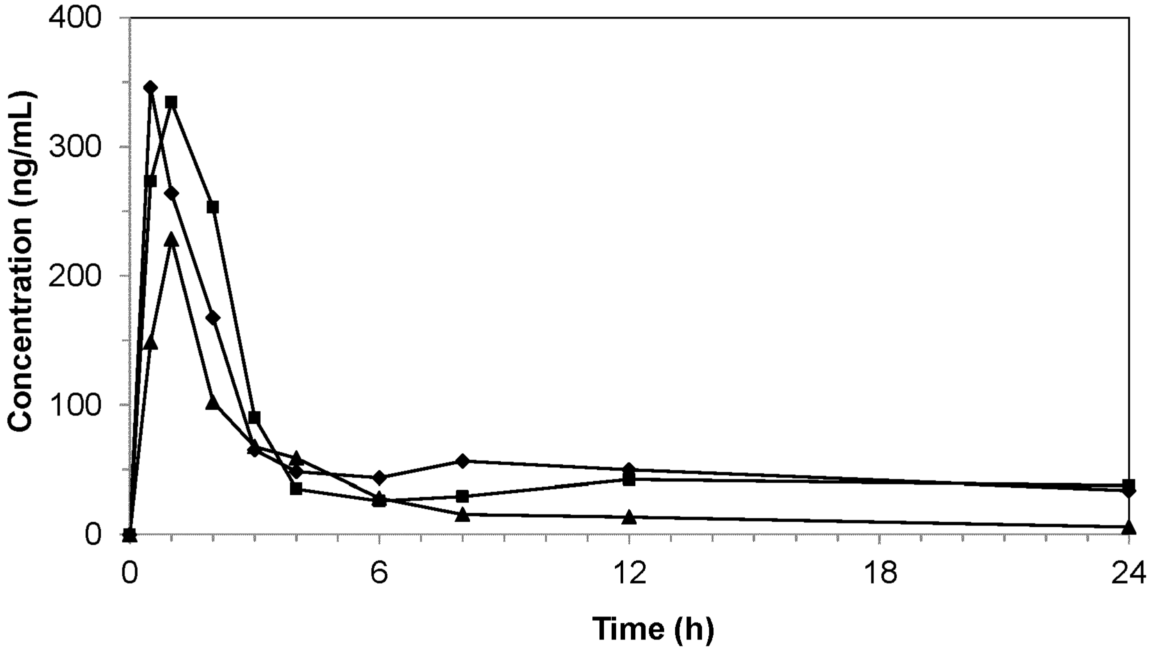
400
300
200
100
0
Concentration (ng/mL)
0
6
12
18
24
Time (h)

FIG. 1B

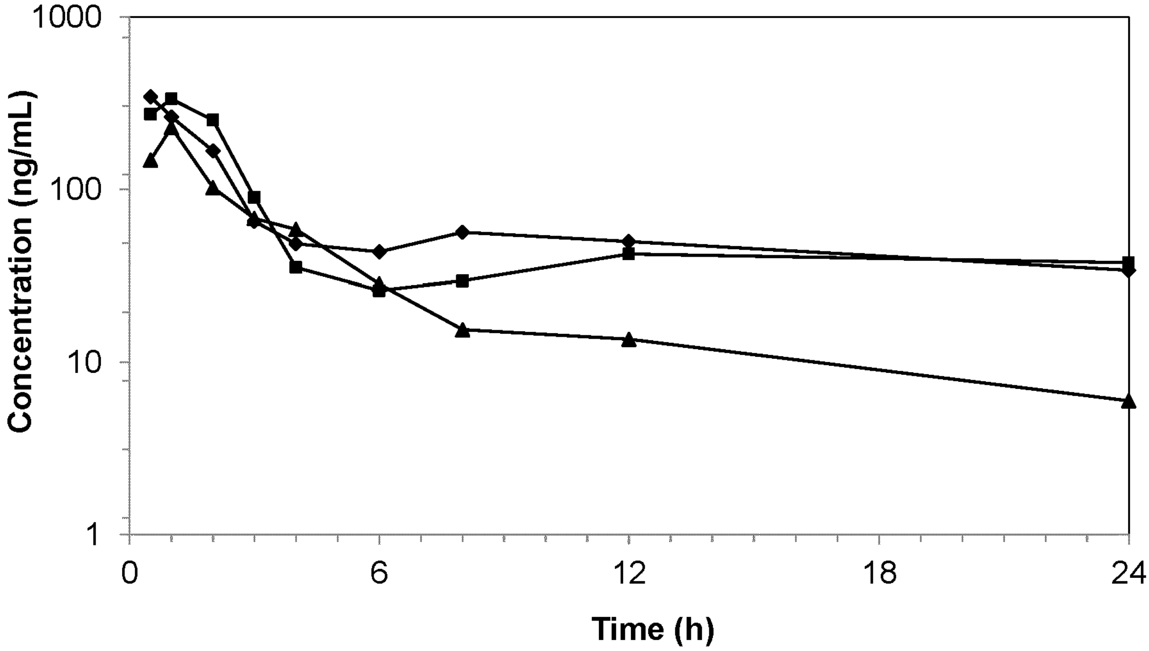
1000
100
10
1
Concentration (ng/mL)
0
6
12
18
24
Time (h)

FIG. 1C

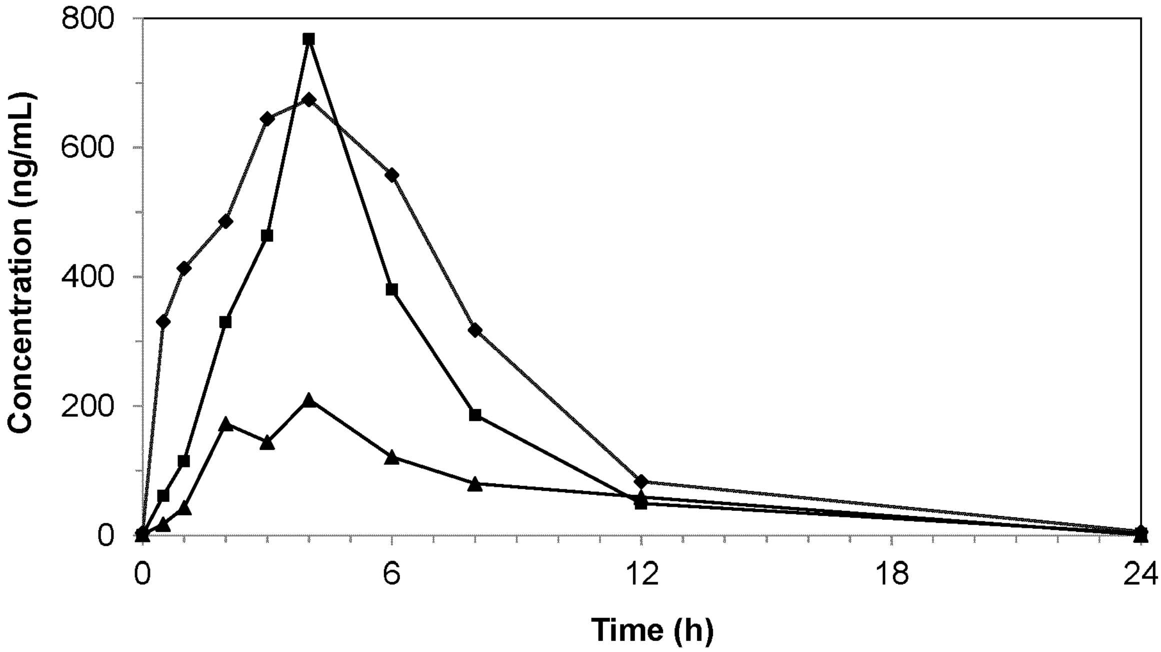
800
600
400
200
0
Concentration (ng/mL)
0
6
12
18
24
Time (h)

FIG. 1D

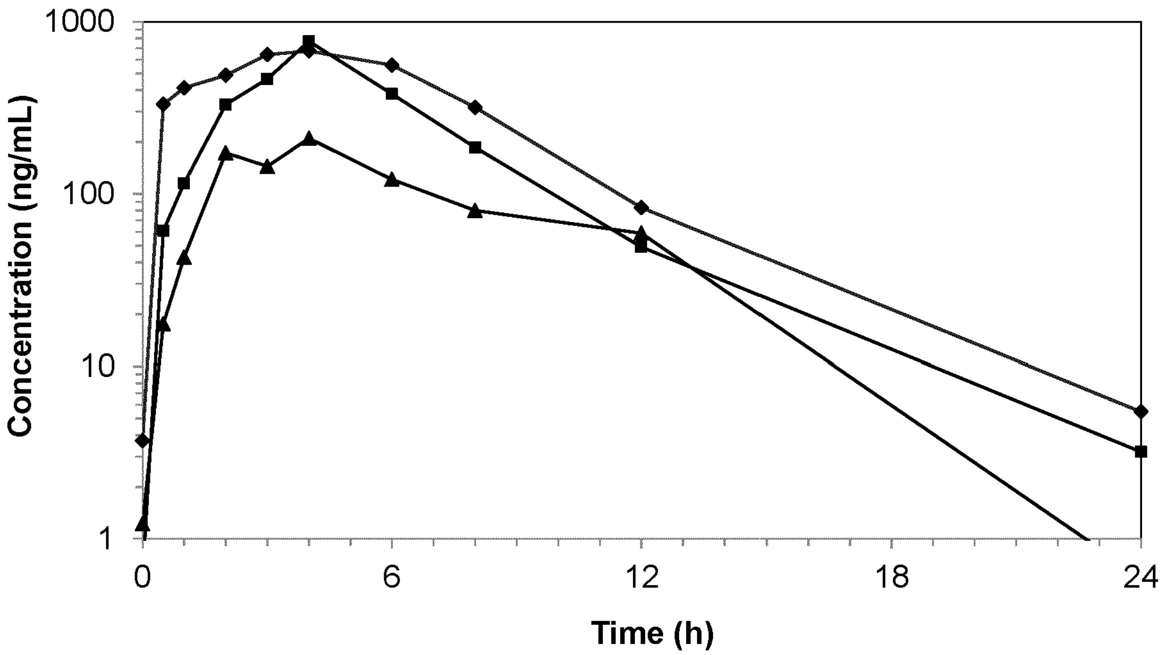
1000
100
10
1
Concentration (ng/mL)
0
6
12
18
24
Time (h)

FIG. 2

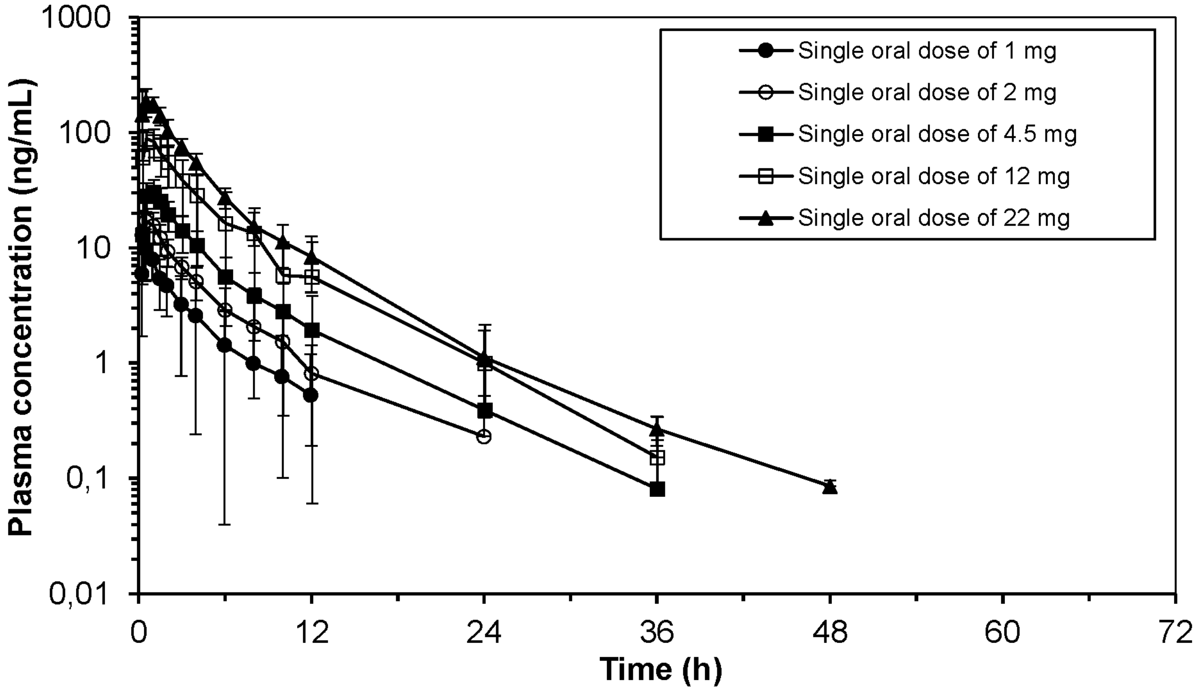
Single oral dose of 1 mg
Single oral dose of 2 mg
Single oral dose of 4.5 mg
Single oral dose of 12 mg
Single oral dose of 22 mg
Plasma concentration (ng/mL)
1000
100
10
1
0,1
0,01
0
12
24
36
48
60
72
Time (h)

FIG. 3

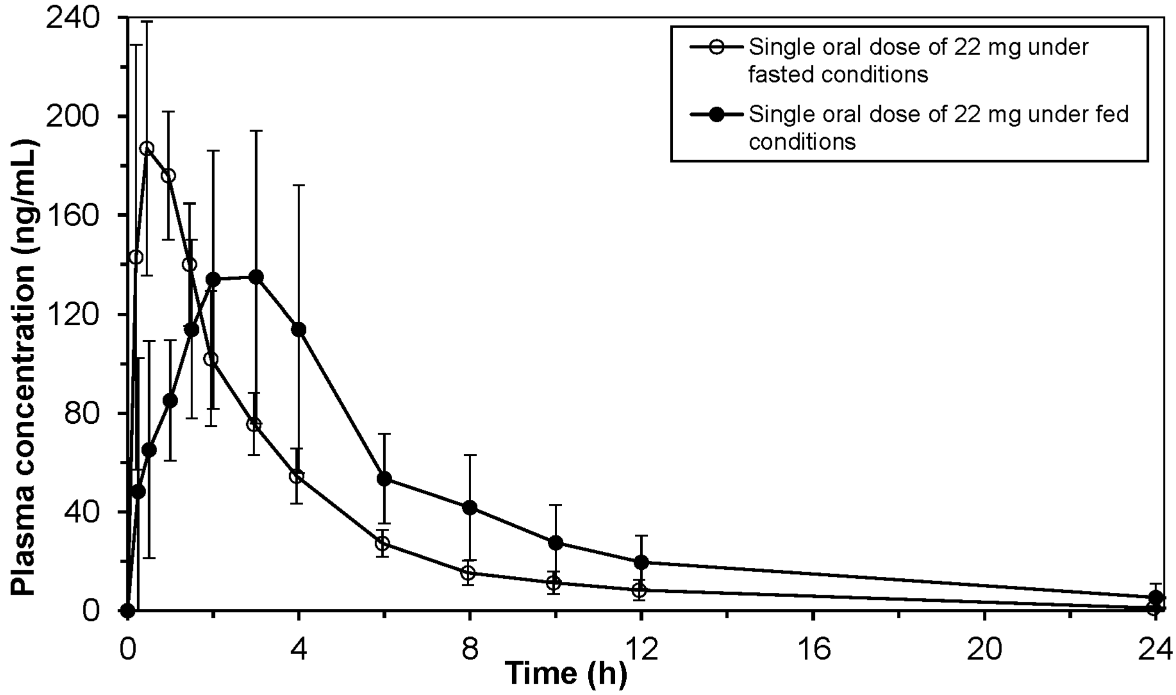
Single oral dose of 22 mg under fasted conditions
Single oral dose of 22 mg under fed conditions
Plasma concentration (ng/mL)
240
200
160
120
80
40
0
0
4
8
12
16
20
24
Time (h)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008055146 A
- WO 2014159637 A
- WO 2010031589 A
- WO 2008116620 A
- WO 200640004 A
- WO 03103671 A
- WO 0387090 A
- WO 0023439 A
- WO 0050418 A
- WO 9964039 A
- WO 9741104 A
- WO 9728153 A
- WO 9707115 A
- WO 9613485 A
- EP 0795547 A
- EP 0796848 A
- EP 0867432 A
- EP 1213289 A
- WO 2019101906 A


### Non-patent literature cited in the description


- *CHEMICAL ABSTRACTS,* 2340111-58-0